# EUROPEAN PATENT APPLICATION

(11) **EP 3 916 016 A1**
(43) Date of publication of application: **01.12.2021**
(21) Application number: 20744779.8
(22) Date of filing: 23.01.2020
(51) Int. Cl.: C07K 16/28, C12N 15/13, C12P 21/02, A61K 39/395, A61P 35/00, A61P 31/00, G01N 33/68

(54) **NOVEL BISPECIFIC ANTIBODY MOLECULE AND BISPECIFIC ANTIBODY SIMULTANEOUSLY COMBINING PD-L1 AND LAG-3**

(30) Priority: 25.01.2019 CN 201910073261
(71) Applicant: Innovent Biologics (Suzhou) Co., Ltd., Suzhou, Jiangsu 215123 (CN)
(72) Inventor: NI, Haiqing, Suzhou, Jiangsu 215123 (CN); CHEN, Bingliang, Suzhou, Jiangsu 215123 (CN); LIU, Junjian, Suzhou, Jiangsu 215123 (CN)
(74) Representative: Ruscoe, Peter James
(86) International application number: PCT/CN2020/073964
(87) International publication number: WO 2020/151762

(57) **Abstract**

The present invention provides a novel, artificially designed antibody molecule comprising:
a polypeptide chain of formula (I):

VH-CH1-Fc-X-VHH;

and
a polypeptide chain of formula (II):

VL-CL;

wherein:
the VH represents a heavy chain variable region;
the CH represents a heavy chain constant region;
the Fc comprises CH2, CH3, and optionally CH4;
the CH1, the CH2, the CH3 and the CH4 represent domains 1, 2, 3 and 4, respectively, of the heavy chain constant region;
the X may be absent, or represents a linker such as a flexible linker when present;
the VHH represents a single-domain antigen-binding site such as a single-domain antibody;
the VL represents a light chain variable region;
the CL represents a light chain constant region;
optionally, a hinge region is present between the CH1 and the Fc.

## Description

### FIELD OF THE INVENTION

The present invention generally relates to the field of immunology and antibody engineering. Specifically, the present invention relates to a novel, artificially designed bispecific antibody molecule, in particular a bispecific antibody simultaneously binding to PD-L1 and LAG-3, a polynucleotide encoding the antibody molecule or individual chains thereof, a vector comprising the polynucleotide, a host cell comprising the polynucleotide or the vector, an immunoconjugate and a pharmaceutical composition comprising the antibody molecule, and use of the antibody molecule in the immunotherapy, prevention and/or diagnosis of a disease.

### BACKGROUND OF THE INVENTION

Antibody molecules capable of targeted specific binding to corresponding antigens thereof are becoming important therapeutic agents, preventive agents and/or diagnostic agents for a variety of diseases such as cancers, autoimmune diseases, inflammatory diseases and infectious diseases. However, monospecific antibodies against a single target have some limitations in clinical applications. Patients may develop resistance or no response after treatment with monospecific antibodies. With researches on cancers and many other diseases, it is recognized that there are often multiple signal transduction pathways involved in the development and progression of diseases, and a single-target immunotherapy is usually less effective in treating many diseases.

Multispecific antibodies such as bispecific antibodies can be designed to act on signal transduction pathways of two or more different mediators simultaneously since they are capable of specifically binding to different antigens. These advantages have expanded the application of multispecific antibodies such as bispecific antibodies.

A large number of imaginative patterns of multispecific antibodies (such as bispecific antibodies) have been developed through antibody engineering and their suitability in therapeutic applications has been studied (Brinkmann U. and Kontermann R. E., The making of bispecific antibodies, Mabs, 2017, 9(2): 182-212).

Multispecific antibodies such as bispecific antibodies can be categorized into many types based on different components and construction methods. For example, based on the substantial bilateral symmetry of multispecific antibody structures, they can be categorized into symmetrical antibodies and asymmetric antibodies; based on the presence or absence of IgG Fc region in multispecific antibodies, they can be categorized into antibody patterns with Fc region and antibody patterns without Fc region; based on the amount of antigen-binding sites in multispecific antibodies, they can be categorized into bivalent, trivalent, or tetravalent antibodies or those of greater valencies, and the like.

Multispecific antibody patterns in the prior art have their own advantages and disadvantages in preparation and application. For example, although blinatumomab can be produced on a large scale using recombinant Chinese hamster ovary (CHO) cells, it is prone to aggregate formation, has a short half-life *in vivo,* and requires an additional continuous infusion device in practice; manufacturing process of catumaxomab is complex and mouse heterologous antibodies are more likely to cause immunogenicity issues in humans.

Accordingly, there is still a need in the art for alternative multispecific antibodies having improved properties, in particular bispecific antibodies. The present invention provides a novel multispecific antibody pattern which is easy to be expressed efficiently in culture cells *in vitro* without complex manufacturing process. At the same time, the bispecific antibody is capable of binding to different antigens simultaneously and retaining the binding activity of each antigen-binding site to the corresponding epitope, as well as other properties. Further, the bispecific antibody pattern disclosed herein is physically and biologically stable, which provides the antibody with better productbility and developability.

### BRIEF SUMMARY

Disclosed herein is a novel bispecific antibody molecule constructed by an antibody engineering technique.

Therefore, in one aspect, the present invention provides a bispecific antibody molecule having one or more of the following characteristics:
(a) specifically binding to one or two antigens with high affinity;
(b) easily expressed in culture cells *in vitro,* and chains of the antibody molecule being capable of correct coupling or pairing;
(c) having good physical stability, in particular, having good long-term thermal stability, and being capable of maintaining biological activities for a long time;
(d) exerting biological functions through regulating (e.g., inhibiting or activating) signaling pathways involving one or more antigens after specifically binding thereto;
(e) exerting effector functions; and
(f) having better anti-tumor activity.

In one embodiment, an antibody molecule disclosed herein comprises a whole antibody portion and a single-domain antibody portion linked to a C-terminus of a heavy chain constant region of the whole antibody portion via a linker.

In one embodiment, the antibody molecule disclosed herein comprises or consists of:
a polypeptide chain of formula (I) (peptide chain #1):

   VH-CH1-Fc-X-VHH;

   and
a polypeptide chain of formula (II) (peptide chain #2):

   VL-CL;

   wherein:
   the VH represents a heavy chain variable region;
   CH represents a domain of a heavy chain constant region;
   the Fc comprises CH2, CH3, and optionally CH4;
   the CH1, the CH2, the CH3 and the CH4 represent domains 1, 2, 3 and 4, respectively, of the heavy chain constant region;
   the X may be absent, or represents a linker such as a flexible linker when present;
   the VHH represents a single-domain antigen-binding site such as a single-domain antibody;
   the VL represents a light chain variable region;
   the CL represents a light chain constant region;
   optionally, a hinge region is further present between the CHI and the Fc.

In one embodiment, the antibody molecule disclosed herein comprises at least one polypeptide chain of formula (I) and one polypeptide chain of formula (II). Preferably, the antibody molecule disclosed herein comprises two (e.g., identical) polypeptide chains of formula (I) and two (e.g., identical) polypeptide chains of formula (II).

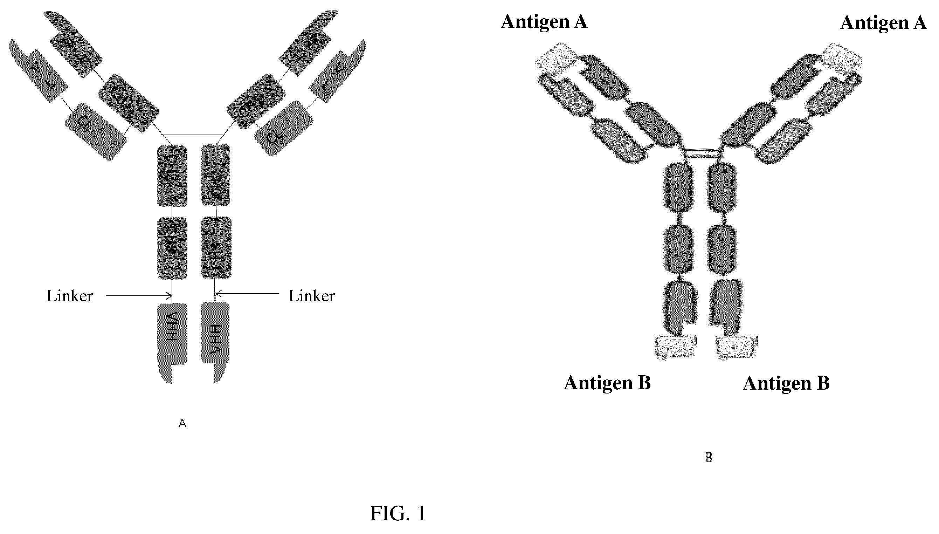

In one embodiment, the structure of the antibody molecule disclosed herein is shown in FIG. 1 (A).

In one embodiment, the antibody molecule or the fragment thereof disclosed herein has 2 or 4 antigen-binding sites, which bind to 2, 3 or 4 different antigens or the same antigen. In one embodiment, the VH in the formula (I) and the VL in the formula (II) form one antigen-binding site, and the VHH in the formula (I) constitutes one antigen-binding site (a single-domain antigen-binding site).

In one embodiment, different antigen-binding sites bind to the same epitope or different epitopes on the same antigen.

In one embodiment, the linker X in the formula (I) in the antibody molecule disclosed herein is a flexible linker, e.g., a linker having glycine and/or serine residues present alone or in combination. In one embodiment, the linker comprises an amino acid sequence (Gly₄Ser)n, wherein n is a positive integer equal to or greater than 1, e.g., n is a positive integer from 1 to 7, such as 2, 3, 4, 5 or 6. In one embodiment, n is 1, 2, 3 or 4.

In one embodiment, the antigen-binding site formed by the VH in the formula (I) and the VL in the formula (II) is human-derived or humanized, or chimeric.

In one embodiment, the single-domain antigen-binding site (VHH) in the antibody molecule disclosed herein is a heavy chain variable domain of an antibody naturally devoid of light chains (e.g., a heavy chain variable domain of a heavy chain antibody naturally existing in a Camelidae species), or a VH-like single domain in an immunoglobulin of fish referred to as a new antigen receptor (NAR) (e.g., IgNAR naturally existing in shark serum), or a recombinant single-domain antigen-binding site derived therefrom (e.g., a camelized human VH domain or a humanized Camelidae antibody heavy chain variable domain). In a preferred embodiment, the single-domain antigen-binding site in the antibody molecule disclosed herein is selected from a heavy chain variable domain of a heavy chain antibody naturally existing in the Camelidae species, a camelized human VH domain, and a humanized Camelidae antibody heavy chain variable domain.

In one embodiment, the VHH molecule in the peptide chain of formula (I) in the antibody molecule disclosed herein may be derived from an antibody produced in Camelidae species (e.g., camel, alpaca, dromedary, llama and guanaco). Species other than Camelidae may also produce heavy chain antibodies naturally devoid of light chains, and VHHs of such heavy chain antibodies are also within the scope of the present invention.

In one embodiment, the CHI and the Fc are from an antibody heavy chain or a derivative thereof.

In one embodiment, the "CH1-Fc" in the formula (I) is in the form of IgG, for example, in the form of IgG1, IgG2 or IgG4. In one embodiment, it is in the form of IgG1. It will be understood that the Fc in the constant domain may be mutated to stabilize the antibody or to enhance the effector function. For example, in a specific embodiment, the effector function is antibody-dependent cell-mediated cytotoxicity (ADCC). In one embodiment, the amino acid mutation is present in the CH2 domain, e.g., the antibody molecule comprises amino acid replacements at positions 234 and 235 (EU numbering, numbered according to the EU index of Kabat et al., Sequences of Proteins of Immunological Interest, 5th Ed., Public Health Service, National Institutes of Health, Bethesda, MD (1991)). In a specific embodiment, the amino acid replacements are L234A and L235A.

In one embodiment, a disulfide bond is present between the CHI and the CL. In one embodiment, if two polypeptide chains of formula (I) are involved, disulfide bonds are present between the hinge regions between CHI and CH2 of the two polypeptide chains of formula (I), and the number of disulfide bonds is variable depending on the IgG form from which the constant domains of the antibody are derived, and in some embodiments, 2 or 4 disulfide bonds are present between the hinge regions.

In one embodiment, the light chain constant domain CL in the formula (II) is from κ or λ.

In one embodiment, a binding site formed by the VH in the formula (I) and the VL in the formula (II) is specific for a first antigen; in one embodiment, the first antigen is LAG-3.

In one embodiment, a binding site formed by the VHH in the formula (I) is specific for a second antigen; in one embodiment, the second antigen is PD-L1.

The type of antigen to which the antibody molecule disclosed herein specifically binds is not particularly limited, and the antigen may be, for example, a cytokine, a growth factor, a hormone, a signaling protein, an inflammatory mediator, a ligand, or a cell surface receptor or a fragment thereof. In one embodiment, the antigen to which the antibody molecule disclosed herein specifically binds is selected from a tumor-associated antigen, an immune checkpoint molecule, an angiogenic factor, a member of a tumor necrosis factor receptor superfamily, a co-stimulatory molecule in immune system, and ligands and/or receptors thereof, such as OX40, CD47, PD1, PD-L1, PD-L2, LAG-3, 4-1BB (CD137), VEGF and GITR.

In one embodiment, the VH-CH1-Fc in the formula (I) constitutes the heavy chain of the whole antibody portion, and the VL-CL of the formula (II) constitutes the light chain of the whole antibody portion.

In one embodiment, the VHH in the formula (II) constitutes a single-domain antibody.

In one embodiment, the antibody disclosed herein also encompasses antigen-binding fragments thereof, such as Fab, Fab', Fab'-SH, Fv, a single chain antibody (e.g., scFv) or (Fab')₂, a single-domain antibody, a diabody (dAb) or a linear antibody.

In some embodiments, a heavy chain and/or light chain of the anti-LAG-3 antibody or the fragment thereof disclosed herein further comprises a signal peptide sequence, such as METDTLLLWVLLLWVPGSTG (SEQ ID NO: 20).

In one aspect, the present invention provides a nucleic acid encoding any one or more polypeptide chains in the antibody molecule disclosed herein, a vector comprising the nucleic acid, and a host cell comprising the nucleic acid or the vector.

In one aspect, the present invention provides a vector comprising a polynucleotide encoding any one or more polypeptide chains in the antibody molecule disclosed herein; preferably, the vector is an expression vector, such as a pTT5 vector.

In one aspect, the present invention provides a method for producing the antibody molecule or the fragment thereof disclosed herein.

In some embodiments, the present invention provides an immunoconjugate, a pharmaceutical composition, a kit, a combination product or an article of manufacture comprising the antibody disclosed herein.

In some embodiments, the antibody, the pharmaceutical composition, the immunoconjugate, the combination product or the kit disclosed herein is used to prevent or treat a disease, such as an autoimmune disease, an inflammatory disease, an infection or a tumor. For example, the disease is a tumor (e.g., cancer) or an infection. In some embodiments, the tumor is a tumor immune escape. Preferably, the tumor is, for example, colon cancer or colorectal cancer or rectal cancer. In another aspect, the present invention relates to a method for preventing or treating a disease in a subject or an individual, wherein the method comprises administering to the subject an effective amount of any of the antibodies or the fragments thereof, the pharmaceutical composition, the immunoconjugate, the combination product or the kit described herein. For example, the disease is a tumor (e.g., cancer) or an infection. In some embodiments, the tumor is a tumor immune escape. In one embodiment, the tumor is, for example, colon cancer or colorectal cancer or rectal cancer. In another aspect, the present invention also relates to use of any of the antibodies or fragments thereof or the immunoconjugate described herein in preparing a medicament, a pharmaceutical composition, a kit or a combination product for the treatment of a tumor (e.g., cancer) or an infection in a subject. In some embodiments, the tumor is a tumor immune escape. In one embodiment, the tumor is, for example, colon cancer or colorectal cancer or rectal cancer.

The present invention also relates to a method for detecting an antigen in a sample.

The present invention also encompasses any combination of the embodiments described herein. Any of the embodiments described herein or any combination thereof is applicable to any and all of the antibodies or the fragments thereof, the immunoconjugate, the pharmaceutical composition, the combination product, the kit, the method and the use described herein.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to accurate arrangement and means of the embodiments shown in the drawings.
FIGs. 1A-1B illustrate the structure of the bispecific antibody disclosed herein.
FIG. 2 shows the purity, as detected by size exclusion chromatography (SEC), of the anti-LAG-3/PD-L1 bispecific antibody IGN-LP prepared according to the present invention.
FIG. 3 shows the binding, as detected by FACS, of the anti-LAG-3/PD-L1 bispecific antibody IGN-LP and an anti-PD-Ll humanized Nb-Fc antibody as a control to CHO cells overexpressing PD-L1 (CHOS-PD-L1). The horizontal axis represents the antibody concentration, and the vertical axis represents the mean fluorescence intensity (MFI).
FIG. 4 shows the binding, as detected by FACS, of the anti-LAG-3/PD-L1 bispecific antibody IGN-LP and an anti-LAG-3 antibody ADI-31853 as a control to 293 cells overexpressing LAG-3 (293-LAG-3). The horizontal axis represents the antibody concentration, and the vertical axis represents the mean fluorescence intensity (MFI).
FIG. 5 shows the simultaneous binding of the anti-LAG-3/PD-L1 bispecific antibody IGN-LP to 293 cells overexpressing LAG-3 (293-LAG-3) and CHO cells overexpressing PD-L1 (CHOS-PD-L1).
FIG. 6 shows the activation of T cells *in vitro* by the bispecific antibody IGN-LP disclosed herein, as well as the anti-LAG-3 antibody ADI-31853, the anti-PD-Ll humanized Nb-Fc antibody, a combination of the two, and IgG as controls, as measured by the concentration of IL-2 produced (pg/mL).
FIG. 7 shows the promotion of the production of TCR:Pmhc conjugate *in vitro,* by the bispecific antibody IGN-LP disclosed herein, as well as the anti-LAG-3 antibody ADI-31853, the anti-PD-Ll humanized Nb-Fc antibody, a combination of the two, and IgG as controls, in a mixture of CHOK1-PD-L1 cells overexpressing PD-L1 (Promega, CS187109) and Jurkat LAG3 overexpressing LAG-3 cells (Promega, CS187109).
FIGs. 8-10 show the inhibition of tumor by the bispecific antibody IGN-LP disclosed herein as well as the anti-LAG-3 antibody ADI-31853, the anti-PD-Ll humanized Nb-Fc antibody, a combination of the two, and huamn IgG as controls.

### DETAILED DESCRIPTION

Unless defined otherwise, all technical and scientific terms used herein have the same meaning as commonly understood by those of ordinary skill in the art to which the present invention belongs. All publications, patent applications, patents, and other references mentioned herein are incorporated by reference in their entireties. In addition, the materials, methods, and examples described herein are illustrative only and are not intended to be limiting. Other features, objectives, and advantages of the present invention will be apparent from the specification and drawings, and from the appended claims.

### Abbreviations

Unless otherwise stated, the abbreviations in this specification have the following meanings:
The following abbreviations are used:
ADCC Antibody-dependent cell-mediated cytotoxicity
CDC Complement-dependent cytotoxicity
CDR Complementarity determining region in a variable region of immunoglobulin
CHO Chinese hamster ovary
EC₅₀ A concentration resulting in 50% potency or binding
K_{D} Equilibrium dissociation constant
ELISA Enzyme-linked immunosorbent assay
FACS Fluorescence-activated cell sorting
MOA Mechanism of action
MLR Mixed lymphocyte reaction
FR Antibody framework region
IC₅₀ A concentration producing 50% inhibition
Ig Immunoglobulin
Kabat An immunoglobulin alignment and numbering system established by Elvin A. Kabat ((1991) Sequences of Proteins of Immunological Interest, 5th Ed., Public Health Service, National Institutes of Health, Bethesda, Md.)
mAb/Mab/MAb Monoclonal antibody
PCR Polymerase chain reaction
IFN Interferon
VL Light chain variable region
VH Heavy chain variable region
LC Light chain
HC Heavy chain
HCDR Heavy chain complementary determining region
LCDR Light chain complementary determining region
IL2 Interleukin-2

### I. Definitions

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more of the options.

The term "comprise" or "include" used herein, unless indicated otherwise, also encompasses the situation where the entirety consists of the described elements, integers or steps. For example, when referring to "comprise" an antibody variable region of a particular sequence, it is also intended to include an antibody variable region consisting of the particular sequence.

The term "antibody" is used herein in the broadest sense, refers to a protein comprising an antigen-binding site, and encompasses natural and artificial antibodies with various structures, including but not limited to monoclonal antibodies, polyclonal antibodies, multispecific antibodies (e.g., bispecific antibodies), single-chain antibodies, intact antibodies, and antibody fragments.

The terms "whole antibody", "full-length antibody", "complete antibody" and "intact antibody" are used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. Each heavy chain constant region consists of 3 domains CH1, CH2 and CH3. Each light chain consists of a light chain variable region (abbreviated herein as VL) and a light chain constant region. Each light chain constant region consists of one domain CL. The VH region and the VL region can be further divided into hypervariable regions (complementarity determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3 and FR4. The constant regions are not directly involved in binding of antibodies to antigens, but exhibit a variety of effector functions.

"Antibody fragment" refers to a molecule different from an intact antibody, which comprises a portion of the intact antibody and binds to an antigen to which the intact antibody binds. Examples of the antibody fragment include, but are not limited to, Fv, Fab, Fab', Fab'-SH, F(ab')2; a diabody; a linear antibody; a single-chain antibody (e.g., scFv); a single-domain antibody; a bivalent or bispecific antibody or a fragment thereof; a Camelidae antibody; and a bispecific antibody or multispecific antibody formed from antibody fragments.

As used herein, the term "epitope" refers to the moiety of an antigen (e.g., human LAG-3 or PD-L1) that specifically interacts with an antibody molecule.

An "antibody that binds to the same or overlapping epitope" as a reference antibody refers to an antibody that blocks 50%, 60%, 70%, 80%, 90% or 95% or more of the binding of the reference antibody to its antigen in a competition assay, or conversely, the reference antibody blocking 50%, 60%, 70%, 80%, 90% or 95% or more of the binding of the antibody to its antigen in a competition assay.

An antibody that competes with a reference antibody for binding to its antigen refers to an antibody that blocks 50%, 60%, 70%, 80%, 90% or 95% or more of the binding of the reference antibody to its antigen in a competition assay. Conversely, the reference antibody blocks 50%, 60%, 70%, 80%, 90% or 95% or more of the binding of the antibody to its antigen in a competition assay. Numerous types of competitive binding assays can be used to determine whether an antibody competes with another, such as direct or indirect solid-phase radioimmunoassay (RIA), direct or indirect solid-phase enzyme immunoassay (EIA), and sandwich competition assay (see, e.g., Stahli et al., 1983, Methods in Enzymology 9:242-253).

An antibody that inhibits (e.g., competitively inhibits) the binding of a reference antibody to its antigen refers to an antibody that inhibits 50%, 60%, 70%, 80%, 90% or 95% or more of the binding of the reference antibody to its antigen. Conversely, the reference antibody inhibits 50%, 60%, 70%, 80%, 90% or 95% or more of the binding of the antibody to its antigen. The binding of an antibody to its antigen can be measured by affinity (e.g., equilibrium dissociation constant). Methods for determining affinity are known in the art, such as ForteBio affinity assay.

An antibody that shows the same or similar binding affinity and/or specificity as a reference antibody refers to an antibody that is capable of having at least 50%, 60%, 70%, 80%, 90% or 95% or more of the binding affinity and/or specificity of the reference antibody. This can be determined by any method known in the art for determining binding affinity and/or specificity.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact site"). CDRs are primarily responsible for binding to antigen epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2 and HCDR3, whereas the CDRs located in the light chain variable domain of the antibody are referred to as LCDR1, LCDR2 and LCDR3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature 342:877-883; Al-Lazikani et al., Standard conformations for the canonical structures of immunoglobulins, Journal of Molecular Biology, 273:927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

For example, according to different CDR determination schemes, the residues of each CDR are as follows.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

CDRs can also be determined based on having the same Kabat numbering positions as a reference CDR sequence (e.g., any of the exemplary CDRs of the present invention).

Unless otherwise stated, the term "CDR" or "CDR sequence" used herein encompasses CDR sequences determined by any one of the schemes above.

Unless otherwise stated, residue positions of an antibody variable region (including heavy chain variable region residues and light chain variable region residues) are numbered according to the Kabat numbering system (Kabat et al., Sequences of Proteins of Immunological Interest, 5th Ed. Public Health Service, National Institutes of Health, Bethesda, Md. (1991)).

In one embodiment, the boundaries of CDRs of the antibody disclosed herein are determined by Kabat rules, IMGT rules, AbM rules, or any combination thereof.

In one embodiment of the present invention, the boundaries of the HCDR1 (positioned at H27-H35B in the Kabat numbering system) of the VH in formula (I) of the antibody molecule disclosed herein are determined by taking Kabat, AbM, Chothia, and experience into consideration, the HCDR2 of the VH is determined by the Kabat rules, the HCDR3 of the VH is determined by the IMGT rules, and the LCDR of the VL in formula (II) is determined by the Kabat rules. In one embodiment of the present invention, the HCDR1 of the VHH in the formula (I) of the antibody molecule disclosed herein is determined by AbM rules, and HCDR2 and HCDR3 are determined by Kabat rules.

Antibodies with different specificities (i.e., different binding sites for different antigens) have different CDRs. However, although CDRs differ from antibody to antibody, only a limited number of amino acid positions within the CDRs are directly involved in antigen binding. The smallest overlapping region can be determined using at least two of the Kabat, Chothia, AbM, and Contact schemes, thereby providing a "minimal binding unit" for antigen binding. The minimal binding unit may be a sub-portion of the CDR. As will be appreciated by those skilled in the art, residues in remaining portions of the CDR sequences can be determined by the structure and protein folding of the antibody. Thus, variants of any CDR presented herein are also considered. For example, in a variant of one CDR, the amino acid residue of the minimal binding unit may remain unchanged, while the remaining CDR residues defined by the Kabat or Chothia may be conservatively substituted.

"Antibody in the form of IgG" refers to the heavy chain constant region of the antibody belonging to the IgG form. Heavy chain constant regions of all antibodies of the same type are identical, and heavy chain constant regions of antibodies of different types are different. For example, an antibody in the form of IgG4 refers to a heavy chain constant region being from IgG4.

The term "single-domain antibody" as used herein generally refers to an antibody comprising only one heavy chain variable region, and having an activity of binding to an antigen, i.e., an antibody comprising only one chain of FR4-VHHCDR3-FR3-VHHCDR2-FR2-VHHCDRI-FRI from C-terminus to N-terminus, which can be from a heavy chain variable domain of a Camelidae heavy chain antibody or a VH-like single-domain of fish IgNAR (v-NAR), and may be produced naturally or by genetic engineering techniques. Examples of single-domain antibodies include single-domain antibodies derived from Camelidae (Llama and camel) and cartilaginous fishes (e.g., nurse sharks) (WO2005/035572).

The term "camelized human VH domain" refers to transferring key elements derived from a Camelidae VHH to a human VH domain, causing that the camelized human VH domain can alone impart antigen-binding specificity without the human VH domain pairing with a VL domain to recognize the target antigen.

The term "binding site" or "antigen-binding site" as used herein refers to a region in an antibody molecule that actually binds to an antigen, which includes VH/VL pairs consisting of light chain variable domains (VL) of antibodies and heavy chain variable domains (VH) of antibodies, heavy chain variable domains derived from Camelidae heavy chain antibodies, VH-like single domains of IgNAR (v-NAR) from sharks, camelized human VH domains, and heavy chain variable domains of humanized Camelidae antibodies. In one embodiment of the present invention, the antibody molecule disclosed herein comprises at least four antigen-binding sites, e.g. two single-domain antigen-binding sites (e.g., VHH) and two antigen-binding sites formed by VH/VL pairs.

The term "single-domain antigen-binding site" refers to a region of an antibody molecule that actually binds to an antigen with a single variable domain (e.g., a heavy chain variable domain (VH)). In one embodiment of the present invention, the single-domain antigen-binding site disclosed herein may constitute a single-domain antibody. In one embodiment of the present invention, the antibody molecule disclosed herein comprises two single-domain antigen-binding sites, which bind to the same or different antigens. In another embodiment of the present invention, the antibody molecule disclosed herein comprises two single-domain antigen-binding sites, which bind to the same or different epitopes.

As used herein, the term "multispecific" antibody refers to an antibody having at least two antigen-binding sites, each of which binds to a different epitope of the same antigen or a different epitope of a different antigen. The antibody provided herein is generally a multispecific antibody, such as a bispecific antibody. A multispecific antibody is an antibody having binding specificities for at least two different epitopes. In one embodiment, provided herein is a bispecific antibody having binding specificities for a first antigen and a second antigen.

The term "immunoglobulin molecule" refers to a protein having a structure of a naturally existing antibody. For example, an IgG is a heterotetrameric glycoprotein of about 150,000 Daltons consisting of two light chains and two heavy chains which are disulfide-bonded. Each immunoglobulin heavy chain has a heavy chain variable region (VH), also called a heavy chain variable domain, followed by three heavy chain constant regions (CH1, CH2, and CH3) from N-terminus to C-terminus. Similarly, each immunoglobulin light chain has a light chain variable region (VL), also called a light chain variable domain, followed by a light chain constant domain (CL) from N-terminus to C-terminus. The heavy chains of an immunoglobulin can be assigned to one of five classes, α (IgA), δ (IgD), ε (IgE), γ (IgG), or µ (IgM), in which some classes can be further divided into subclasses such as γ (IgG1), γ₂ (IgG2), γ₃ (IgG₃), γ₄ (IgG₄), α₁ (IgA₁), and α₂ (IgA₂). The light chains of an immunoglobulin can be divided into one of two categories, κ or λ, based on the amino acid sequence of constant domains thereof. An immunoglobulin consists essentially of two Fab molecules and one Fc domain linked by an immunoglobulin hinge region.

The term "effector function" refers to biological activities attributed to an immunoglobulin Fc region that vary with immunoglobulin isotype. Examples of immunoglobulin effector functions include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down regulation of cell surface receptors (such as B-cell receptors), and B-cell activation.

The term "chimeric antibody" is an antibody molecule in which: (a) a constant region or a portion thereof is modified, substituted, or exchanged such that antigen-binding sites are linked to constant regions of different or modified classes, effector functions and/or species, or disparate molecules imparting new properties (e.g., enzymes, toxins, hormones, growth factors, and drugs) to chimeric antibodies, etc.; or (b) a constant region or a portion thereof is modified, substituted, or exchanged by variable regions with different or modified antigen-binding specificities. For example, a mouse antibody can be modified by substituting its constant region with a constant region from a human immunoglobulin. Due to the substitution with a human constant region, the chimeric antibody can retain its specificity for recognizing antigens, while having reduced antigenicity in humans as compared to the original mouse antibody.

"Humanized" antibody is an antibody that retains the antigen-specific reactivity of a non-human antibody (such as a mouse monoclonal antibody) and has lower immunogenicity when administered to humans as a therapeutic agent. This can be achieved, for example, by retaining non-human antigen-binding sites and substituting the remainder of the antibodies with their human counterparts (i.e., the portions of the constant and variable regions not involved in binding are the corresponding parts of human antibodies). See, e.g., Padlan, Anatomy of the antibody specimen, Mol. Immun., 1994, 31:169-217. Other examples of human antibody engineering techniques include, but are not limited to, the Xoma technology disclosed in US 5,766,886.

"Human antibody" refers to an antibody having an amino acid sequence which corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen-binding residues.

The term "...valent" antibody refers to the number of antigen-binding sites present in an antibody molecule. "Bivalent", "trivalent", and "tetravalent" antibodies refer to the presence of 2 antigen-binding sites, 3 antigen-binding sites, and 4 antigen-binding sites in an antibody molecule, respectively. In one embodiment, the bispecific antibody reported herein is "tetravalent".

The term "flexible linker" or "linker" refers to a linker peptide consisting of amino acids, such as glycine and/or serine residues used alone or in combination, to link various variable domains in an antibody. In one embodiment, the flexible linker is a Gly/Ser linker peptide comprising an amino acid sequence (Gly₄Ser)n, wherein n is a positive integer equal to or greater than 1, for example, a positive integer from 1 to 7, such as 2, 3 or 4. In one embodiment, the flexible linker is (Gly₄Ser)₂ (SEQ ID NO: 5). Also included within the scope of the present invention is the linker described in WO2012/138475, which is incorporated herein by reference.

As used herein, the term "binding" or "specific binding" means that the binding effect is selective for antigens and can be distinguished from unwanted or non-specific interactions. The ability of an antigen-binding site to bind to a particular antigen can be determined by an enzyme-linked immunosorbent assay (ELISA) or a conventional binding assay known in the art.

The term "antigen" refers to a molecule that induces an immune response. Such an immune response may involve antibody production or activation of specific immune cells, or both. Those skilled will understand that any macromolecules, including essentially all proteins or peptides, can be used as antigens. In addition, an antigen may be derived from recombinant or genomic DNA. In some embodiments described herein, the first antigen and the second antigen are two different antigens.

The terms "tumor-associated antigen" and "cancer antigen" can be used interchangeably to refer to molecules (generally proteins, carbohydrates, or lipids) preferably expressed completely or as fragments (e.g., MHC/peptide) on the surface of cancer cells, compared to normal cells, and the molecules can be used in the preferential targeting of cancer cells by the pharmaceutical agent. In some embodiments, the tumor-associated antigen is a cell surface molecule overexpressed in tumor cells compared to normal cells, such as 1-fold overexpression, 2-fold overexpression, 3-fold overexpression, or more fold overexpression compared to normal cells. In some embodiments, the tumor-associated antigen is a cell surface molecule inappropriately synthesized in tumor cells, such as a molecule comprising deletions, additions or mutations compared to molecules expressed on normal cells. In some embodiments, the tumor-associated antigen is expressed completely or as fragments only on the surface of tumor cells, and is not synthesized or expressed on the surface of normal cells.

The term "cytokine" is a generic term for proteins that are released by a cell population and act as intercellular mediators on another cell. As used herein, the term "cytokine" includes proteins from natural sources or from recombinant cell cultures and biologically active equivalents of native sequence cytokines, including small molecule entities produced by artificial synthesis, and pharmaceutically acceptable derivatives and salts thereof.

"Immunoconjugate" is an antibody conjugated to one or more other substances, including but not limited to cytotoxic agents or labels.

The term "lymphocyte-activation gene-3" or "LAG-3" includes all isotypes, mammal (e.g., human) LAG-3, a species homolog of human LAG-3, and an analog comprising at least one common epitope of LAG-3. The amino acid and nucleotide sequences of LAG-3 (e.g., human LAG-3) are known in the art, for example, see Triebel et al., (1990) J. Exp. Med. 171: 1393-1405. In some embodiments, the term "human LAG-3" refers to human sequence LAG-3, such as the complete amino acid sequence of human LAG-3 having Genbank accession No. NP_002277. LAG-3 is also known in the art as, for example, CD223. The human LAG-3 sequence may differ from human LAG-3 having Genbank accession No. NP_002277, e.g., by conservative mutations or mutations in non-conserved regions, and the LAG-3 has substantially the same biological functions as the human LAG-3 having Genbank accession No. NP_002277. For example, a biological function of human LAG-3 is to have an epitope in the extracellular domain of LAG-3 to which the antibody disclosed herein specifically binds, or a biological function of human LAG-3 is to bind to an MHC class II molecule.

The term "anti-LAG-3 antibody", "anti-LAG-3", "LAG-3 antibody", or "LAG-3-binding antibody" as used herein refers to an antibody that can bind to LAG-3 protein or a fragment thereof with sufficient affinity. In one embodiment, the anti-LAG-3 antibody binds to a non-LAG-3 protein to an extent less than about 10%, about 20%, about 30%, about 40%, about 50%, about 60%, about 70%, about 80% or about 90% or more of the binding of the antibody to LAG-3, as measured, for example, by radioimmunoassay (RIA), biological optical interferometry, or MSD assay.

The terms "programmed cell death 1 ligand 1", "PD-L1", "programmed death ligand 1", "cluster of differentiation 274", "CD274", or "B7 homolog 1" as used herein refer to any natural PD-L1 from any vertebrate source including mammals such as primates (e.g., humans) and rodents (e.g., mice and rats). The terms encompass "full length", unprocessed PD-L1, and PD-L1 of any form that results from processing in the cell. PD-L1 may present as a transmembrane protein or as a soluble protein. The terms also encompass variants of naturally occurring PD-L1, such as splicing variants or allelic variants. The basic structure of PD-L1 includes four domains: an extracellular Ig-like V-type domain and an Ig-like C2-type domain, a transmembrane domain and a cytoplasmic domain. Additional information about the human PD-L1 gene including genomic DNA sequences can be found under NCBI Gene ID No. 29126. Additional information about the human PD-L1 gene including genomic DNA sequences can be found under NCBI Gene ID No. 60533. The amino acid sequence of an exemplary full-length human PD-L1 protein can be found, e.g., under NCBI accession number NP_001254653 or UniProt accession number Q9NZQ7, and the sequence of an exemplary full-length mouse PD-L1 protein can be found, e.g., under NCBI accession number NP_068693 or UniProt accession number Q9EP73.

The term "anti-PD-Ll antibody", "anti-PD-L1", "PD-L1 antibody", or "PD-L1-binding antibody" as used herein refers to an antibody that can bind to PD-L1 protein or a fragment thereof with sufficient affinity. In one embodiment, the anti-PD-Ll antibody binds to a non-PD-Ll protein to an extent less than about 10%, about 20%, about 30%, about 40%, about 50%, about 60%, about 70%, about 80% or about 90% or more of the binding of the antibody to PD-L1, as measured, for example, by radioimmunoassay (RIA), biological optical interferometry, or MSD assay.

The term "inhibitor" or "antagonist" includes substances that reduce certain parameters (e.g., activity) of a given molecule. For example, this term includes substances that inhibit the activity (e.g., LAG-3 or PD-L1 activity) of a given molecule by at least 5%, 10%, 20%, 30%, 40% or more. Therefore, the inhibitory effect needs not be 100%.

"Functional Fc region" possesses the "effector functions" of Fc regions of native sequences. Exemplary "effector functions" include C1q binding, CDC, Fc receptor binding, ADCC, phagocytosis, cell surface receptor (e.g., B cell receptor, or BCR) down-regulation, and the like. Such effector functions generally require that the Fc region is associated with a binding domain (e.g., an antibody variable domain) and can be assessed using a variety of assays, such as those disclosed herein.

"Effector function" refers to biological activities which can be attributed to the antibody Fc region and vary with the antibody isotype. Examples of antibody effector functions include: C1q binding and complement-dependent cytotoxicity (CDC), Fc receptor binding, antibody-dependent cell-mediated cytotoxicity (ADCC), phagocytosis, cell surface receptors (such as B cell receptors) down-regulation, and B cell activation.

"Human effector cell" refers to a leukocyte that expresses one or more FcRs and executes effector functions. In certain embodiments, the cell expresses at least Fc effectors and executes effector functions of ADCC. Examples of human leukocytes mediating ADCC include peripheral blood mononuclear cells (PBMC), natural killer (NK) cells, monocytes, cytotoxic T cells, and neutrophils. Effector cells can be isolated from their natural sources, such as blood.

The term "effective amount" refers to an amount or dosage of the antibody, fragment, conjugate or composition disclosed herein which generates expected effects in a patient in need of treatment or prevention after administered to the patient in a single or multiple doses. The effective amount can be easily determined by an attending physician as a person skilled in the art by considering a variety of factors as follows: species such as mammals; its size, age, and general health condition; the specific disease involved; the extent or severity of the disease; response in an individual patient; specific antibody administered; route of administration; bioavailability characteristics of the administered preparation; selected dosage regimen; and use of any concomitant therapy.

The "therapeutically effective amount" refers to an amount effective to achieve a desired therapeutic result at a necessary dosage for a necessary period of time. The therapeutically effective amount of an antibody or antibody fragment, or conjugate or composition thereof may vary depending on a variety of factors such as disease state, age, sex, and weight of an individual, and the ability of the antibody or antibody portion to elicit a desired response in an individual. The therapeutically effective amount is also such an amount that any toxic or undesired effect of the antibody or the fragment thereof, or conjugate or composition thereof is inferior to the therapeutically beneficial effect. The "therapeutically effective amount" inhibits a measurable parameter (e.g., tumor growth rate) by preferably at least about 20%, more preferably at least about 40%, even more preferably at least about 50%, 60%, or 70%, and still more preferably at least about 80%, relative to untreated subjects. The ability of a compound to inhibit a measurable parameter (e.g., cancer) can be evaluated in an animal model system that predicts efficacy in human tumors. Optionally, such property of a composition can be evaluated by examining the inhibition ability of the compound, which can be measured *in vitro* by assays known to those skilled.

The "prophylactically effective amount" refers to an amount effective to achieve a desired prophylactic result at a necessary dosage for a necessary period of time. Generally, since a prophylactic dose is administered in a subject before or at an earlier stage of a disease, a prophylactically effective amount will be less than a therapeutically effective amount.

A "Fab" fragment includes a heavy chain variable domain and a light chain variable domain, and also includes the constant domain of the light chain and the first constant domain (CHI) of the heavy chain. A Fab' fragment differs from the Fab fragment due to addition of some residues (including one or more cysteine from an antibody hinge region) to the carboxyl terminal of the heavy chain CHI domain. A Fab'-SH refers to a Fab' in which the cysteine residue of the constant domain carries a free thiol group. An F(ab')₂ antibody fragment was originally generated as paired Fab' fragments with hinge cysteines between the Fab' fragments. Other chemical couplings of antibody fragments are also known.

The term "Fc region" is used herein to define a C-terminus region of an immunoglobulin heavy chain, which comprises at least one portion of a constant region. The "Fc region" includes Fc regions of native sequences and variant Fc regions. In certain embodiments, a human IgG heavy chain Fc region extends from Cys226 or Pro230 to the carbonyl end of the heavy chain. However, the C-terminal lysine (Lys447) of the Fc region may or may not be present. Unless otherwise stated, the numbering of amino acid residues in the Fc region or constant region is based on an EU numbering system, which is also called EU index, as described in Kabat et al., Sequences of Proteins of Immunological Interest, 5th ed. Public Health Service, National Institutes of Health, Bethesda, MD, 1991.

The term "variable region" or "variable domain" refers to a domain of a heavy chain or light chain of an antibody involved in the binding of the antibody to an antigen. Variable domains of heavy chains and light chains of native antibodies often have similar structures, wherein each domain contains four conserved framework regions (FRs) and three complementarity determining regions (CDRs). (See, e.g., Kindt et al., Kuby Immunology, 6th Ed., W.H. Freeman and Co., p 91 (2007)). A single VH or VL domain may be sufficient to provide antigen-binding specificity. In addition, a VH or VL domain from an antibody binding to a particular antigen can be used to isolate antibodies that bind to the antigen, so as to screen libraries of complementary VL or VH domains. See, e.g., Portolano et al., J. Immunol., 150:880-887 (1993); Clarkson et al., Nature, 352:624-628 (1991).

The term "host cell" refers to a cell into which an exogenous polynucleotide has been introduced, including progeny of such cells. Host cells include "transformants" and "transformed cells", which include primary transformed cells and progeny derived therefrom, regardless of the number of passages. Progeny may not be exactly the same as parent cells in terms of nucleic acid content, and may contain mutations. Mutant progeny having the same function or biological activities that are screened or selected from the initially transformed cells are included herein. Host cells are any type of cell systems that can be used to produce the antibody molecule disclosed herein, including eukaryotic cells, e.g., mammalian cells, insect cells, and yeast cells; and prokaryotic cells, e.g., *E. coli* cells. Host cells include cultured cells, as well as cells within a transgenic animal, a transgenic plant, or a cultured plant tissue or animal tissue.

The term "anti-tumor effect" refers to a biological effect that can be demonstrated by a variety of means, including but not limited to, for example, decrease in tumor volume, decrease in number of tumor cells, decrease in tumor cell proliferation, or decrease in tumor cell viability.

The terms "tumor" and "cancer" are used interchangeably herein and encompass solid and liquid tumors.

The terms "cancer" and "cancerous" refer to or describe a physiological disease in mammals that is typically characterized by unregulated cell growth.

The term "tumor" refers to all neoplastic cell growth and proliferation, whether being malignant or benign, and all pre-cancerous and cancerous cells and tissues. The terms "cancer", "cancerous" and "tumor" are not mutually exclusive when referred to herein.

"Tumor immune escape" refers to tumors evading immune recognition and clearance. As such, as a therapeutic concept, tumor immunity is "treated" when such evasion diminishes, and the tumor is recognized and attacked by the immune system. Examples of tumor recognition include tumor binding, tumor shrinkage and tumor clearance.

The term "label" used herein refers to a compound or composition which is directly or indirectly conjugated or fused to an agent, such as a polynucleotide probe or an antibody, and facilitates the detection of the agent to which it is conjugated or fused. The label itself can be detectable (e.g., a radioisotope label or a fluorescent label) or can catalyze a chemical change to a detectable substrate compound or composition in the case of enzymatic labeling. The term is intended to encompass direct labeling of a probe or an antibody by coupling (i.e., physical linking) a detectable substance to the probe or the antibody and indirect labeling of a probe or an antibody by reacting with another reagent which is directly labeled. Examples of indirect labeling include detection of a primary antibody using a fluorescently labeled secondary antibody, and end labeling of a biotinylated DNA probe, such that it can be detected with a fluorescently labeled streptavidin.

"Individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human.

An "isolated" antibody is an antibody which has been separated from components of its natural environment. In some embodiments, the antibody is purified to a purity greater than 95% or 99% as determined by, e.g., electrophoresis (e.g., SDS-PAGE, isoelectric focusing (IEF) and capillary electrophoresis) or chromatography (e.g., ion exchange or reverse-phase HPLC). For a review of methods for assessing antibody purity, see, e.g., Flatman et al., J. Chromatogr., B848:79-87 (2007).

An "isolated" nucleic acid is a nucleic acid molecule which has been separated from components of its natural environment. The isolated nucleic acid includes a nucleic acid molecule contained in a cell that typically comprises the nucleic acid molecule, but the nucleic acid molecule exists extrachromosomally or at a chromosomal location that is different from its natural chromosomal location.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needlema and Wunsch algorithm ((1970) J. Mol. Biol., 48:444-453; available at http://www.gcg.com) which has been integrated into the GAP program of the GCG software package, using the Blossom 62 matrix or PAM250 matrix and gap weight of 16, 14, 12, 10, 8, 6, or 4 and length weight of 1, 2, 3, 4, 5, or 6. In another preferred embodiment, the percent identity between two nucleotide acid sequences is determined with the GAP program of the GCG software package (available at http://www.gcg.com), using the NWSgapdna.CMP matrix and gap weight of 40, 50, 60, 70, or 80 and length weight of 1, 2, 3, 4, 5, or 6. A particularly preferred parameter set (and one that should be used unless otherwise stated) is a Blossom 62 scoring matrix with a gap penalty of 12, a gap extension penalty of 4, and a frameshift gap penalty of 5.

The percent identity between two amino acid sequences or nucleotide sequences can also be determined with PAM120 weighted remainder table, gap length penalty of 12 and gap penalty of 4, using the E. Meyers and W. Miller algorithm which has been incorporated into the ALIGN program (version 2.0) ((1989) CABIOS, 4:11-17).

Additionally or alternatively, the nucleic acid sequences and protein sequences described herein can be further used as "query sequences" to perform searches against public databases to, e.g., identify other family member sequences or related sequences.

As used herein, the term "hybridization under conditions of low stringency, medium stringency, high stringency, or extreme stringency" describes hybridization and washing conditions. Instructions for performing hybridization reactions can be found in Current Protocols in Molecular Biology, John Wiley & Sons, N.Y. (1989), 6.3.1-6.3.6, which is incorporated by reference. Aqueous and non-aqueous methods are described in the references and either method can be used. The specific hybridization conditions mentioned herein are as follows: 1) low stringency hybridization conditions are in 6× sodium chloride/sodium citrate (SSC) at about 45 °C, followed by two washes in 0.2× SSC, 0.1% SDS at least at 50 °C (for low stringency conditions, the temperature of the washes can be increased to 55 °C); 2) medium stringency hybridization conditions are in 6× SSC at about 45 °C, followed by one or more washes in 0.2× SSC, 0.1% SDS at about 60 ° C; 3) high stringency hybridization conditions are in 6× SSC at about 45 °C, followed by one or more washes in 0.2× SSC, 0.1% SDS at 65 °C; and preferably 4) extreme stringency hybridization conditions are in 0.5 M sodium phosphate, 7% SDS at 65 °C, followed by one or more washes in 0.2× SSC, 0.1% SDS at 65 °C. Extreme stringency condition (4) is a preferred condition and the one that should be used unless otherwise stated.

The term "pharmaceutical composition" refers to such a composition that exists in a form allowing effective biological activity of the active ingredient contained therein, and does not contain additional ingredients having unacceptable toxicity to a subject to which the composition is administered.

The term "pharmaceutical supplementary material" refers to diluents, adjuvants (e.g., Freund's adjuvants (complete and incomplete)), carriers, excipients or stabilizers, etc., which are co-administered with active substance.

As used herein, "treatment" (or "treat" or "treating") refers to slowing, interrupting, arresting, alleviating, stopping, reducing, or reversing the progression or severity of an existing symptom, disorder, condition, or disease. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and improving prognosis. In some embodiments, the antibody molecule disclosed herein is used to delay the progression of a disease or to slow the progression of a disease.

As used herein, "prevention" (or "prevent" or "preventing") includes the inhibition of the onset or progression of a disease or disorder, or a symptom of a specific disease or disorder. In some embodiments, subjects with family history of cancer are candidates for preventive regimens. Generally, in the context of cancer, the term "prevention" refers to the administration of a drug prior to the onset of signs or symptoms of a cancer, particularly in subjects at risk of cancer.

The term "therapeutic agent" described herein encompasses any substance effective in preventing or treating diseases, such as tumors (e.g., cancer) and infections, including chemotherapeutic agents, cytotoxic agents, vaccines, additional antibodies, anti-infective active agents, small molecule drugs, or immunomodulatory agents.

"Chemotherapeutic agents" include chemical compounds useful in treatment of cancer.

The term "immunomodulatory agent" used herein refers to a natural or synthetic active agent or drug that suppresses or modulates an immune response. The immune response may be a humoral response or a cellular response.

The term "small molecule drug" refers to a low molecular weight organic compound capable of regulating biological processes.

As used herein, the term "cytotoxic agent" refers to a substance that inhibits or prevents cell function and/or causes cell death or destruction.

The term "anti-infective active agent" includes any molecule that specifically inhibits or eliminates the growth of microorganisms such as viruses, bacteria, fungi, or protozoa, e.g., parasites, and is not lethal to the host, at the administration concentration and interval of administration. As used herein, the term anti-infective active agent includes antibiotics, antibacterials, antivirals, antifungals, and antiprotozoals. In one specific aspect, the anti-infective active agent is non-toxic to the host at the administration concentration and interval of administration.

Antibacterial anti-infective active agents or antibacterials can be broadly classified into bactericidal (i.e., directly killing) or bacteriostatic (i.e., preventing division). Antibacterial anti-infective active agents can be further classified into narrow-spectrum antibacterial agents (i.e., affecting only limited bacterial subtypes, such as Gram-negative, etc.) or broad-spectrum antibacterial agents (i.e., affecting a wide range of species). The term "antiviral" includes any substance that inhibits or eliminates the growth, pathogenicity, and/or survival of a virus. The term "antifungal" includes any substance that inhibits or eliminates the growth, pathogenicity, and/or survival of a fungus. The term "antiprotozoal" includes any substance that inhibits or eliminates the growth, pathogenicity, and/or survival of protozoan organisms (e.g., parasites).

The term "combination product" refers to a kit with a fixed combination, a non-fixed combination, or a part for combined administration in the form of a dose unit, wherein two or more therapeutic agents can be independently administered simultaneously or separately administered at certain intervals of time, especially when these intervals allow combination partners to exhibit collaboration, such as synergistic effect. The term "fixed combination" means that the antibody disclosed herein and a combination partner (e.g., other therapeutic agents) are simultaneously administered to a patient in the form of a single entity or dose. The term "non-fixed combination" means that the antibody disclosed herein and a combination partner (e.g., other therapeutic agents) are simultaneously, concurrently, or sequentially administered to a patient as separate entities, without specific time limitation, wherein such administration provides therapeutically effective levels of the two therapeutic agents in the patient. The latter is also applicable to a cocktail therapy, e.g., administration of three or more therapeutic agents. In one preferred embodiment, the drug combination is a non-fixed combination.

The term "combination therapy" or "combined therapy" means that two or more therapeutic agents are administered to treat a cancer or an infection as described herein. Such administration includes co-administration of these therapeutic agents in a substantially simultaneous manner, for example, in a single capsule with a fixed proportion of active ingredients. Alternatively, such administration includes co-administration or separate administration or sequential administration of active ingredients in a variety of or separate containers (such as tablets, capsules, powder and liquid). The powder and/or liquid can be reconstituted or diluted to a desired dosage before administration. In some embodiments, the administration also includes using each type of therapeutic agents in a sequential manner at approximately the same time or at different times. In any case, the therapeutic regimen will provide the beneficial effect of the drug combination in the treatment of disorders or symptoms described herein.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operatively linked. Such vectors are called "expression vectors" herein.

"Subject/patient sample" refers to a collection of cells or fluids obtained from a patient or a subject. The source of tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development. Tissue samples may comprise compounds which are naturally not mixed with tissues, such as preservatives, anticoagulants, buffers, fixatives, nutrients, antibiotics, and the like. Examples of tumor samples include but are not limited to tumor biopsies, fine needle aspirates, bronchial lavage fluids, pleural fluids, sputa, urine, surgical specimens, circulating tumor cells, serum, plasma, circulating plasma proteins, ascites, primary cell cultures or cell lines derived from tumors or exhibiting tumor-like properties, and preserved tumor samples such as formalin-fixed, paraffin-embedded tumor samples or frozen tumors samples.

The term "package insert" is used to refer to the instructions generally contained in the commercial package of therapeutic products, which contain information about indications, usage, dosage, administration, combination therapies, contraindications and/or warnings related to the application of such therapeutic products.

### II. Antibody molecule disclosed herein

The present invention provides a novel antibody molecule that can be used for immunotherapy, prevention and/or diagnosis of a variety of diseases. The antibody molecule disclosed herein comprises at least 2, 3 or 4 antigen-binding sites, and can function as a monospecific antibody, a bispecific antibody or a multispecific antibody, and preferably, function as a bispecific antibody.

In one embodiment, the single-domain antigen-binding site (VHH) in the formula (I) in the antibody molecule disclosed herein is a single heavy chain variable domain capable of specifically binding to a target antigen epitope with high affinity, e.g., a heavy chain variable domain derived from a Camelidae heavy chain antibody, a v-NAR of IgNAR from sharks, a camelized human VH domain, a humanized Camelidae antibody heavy chain variable domain, and a recombinant single domain thereof. In one embodiment, the single-domain antigen-binding site in the antibody molecule disclosed herein is a heavy chain variable domain derived from a Camelidae heavy chain antibody, a camelized human VH domain and/or a humanized Camelidae antibody heavy chain variable domain. The size, structure and antigenicity for human subjects of antibody proteins obtained from Camelidae species (such as camel, alpaca, dromedary, llama and guanaco) have been characterized in the prior art. Certain natural IgG antibodies from the Camelidae mammalian family lack light chains and are therefore structurally different from a common four-chain antibody structure with two heavy and two light chains of other animals. See PCT/EP 93/02214 (WO94/04678 published on March 3, 1994).

A heavy chain variable domain VHH of a Camelidae heavy chain antibody that has high affinity for target antigens can be obtained by genetic engineering. See, e.g., U.S. Patent No. 5,759,808 issued on June 2, 1998. Similar to other non-humanized antibody fragments, amino acid sequences of Camelidae VHHs can be altered by recombination to obtain sequences that more closely mimic a human sequence, i.e., "humanized", thereby reducing the antigenicity of the Camelidae VHHs for humans. In addition, key elements derived from Camelidae VHHs can also be transferred to human VH domains to obtain camelized human VH domains. A VHH has a molecular weight that is one-tenth of that of a human IgG molecule, and has a physical diameter of only a few nanometers. A VHH itself has extremely high thermal stability, stability against extreme pH and protease digestion, and low antigenicity. Therefore, in one embodiment of the antibody molecule disclosed herein, the VHH in the formula (I), as a building block, contributes to the stability and the low antigenicity for human subjects of the antibody molecule disclosed herein.

In one embodiment, the VHH in the formula (I) of the antibody molecule disclosed herein specifically binds to PD-L1 (e.g., human PD-L1).

In one embodiment, the VHH in the formula (I) of the antibody molecule disclosed herein that specifically binds to PD-L1 comprises:
(i) three complementarity determining regions (VHH CDRs) in SEQ ID NO: 6; or
(ii) relative to the sequence of (i), a sequence comprising a total of at least one and no more than 5, 4, 3, 2, or 1 amino acid alteration (preferably amino acid replacement, and more preferably conservative replacement) in the three CDRs.

In a preferred embodiment, the VHH in the formula (I) of the antibody molecule disclosed herein that specifically binds to PD-L1 comprises:
complementarity determining regions (CDRs) VHH CDR1, VHH CDR2 and VHH CDR3, wherein the VHH CDR1 comprises or consists of an amino acid sequence set forth in SEQ ID NO: 10, or the VHH CDR1 comprises an amino acid sequence having one, two or three alterations (preferably amino acid replacements, and more preferably conservative replacements) compared with an amino acid sequence set forth in SEQ ID NO: 10; the VHH CDR2 comprises or consists of an amino acid sequence set forth in SEQ ID NO: 11, or the VHH CDR2 comprises an amino acid sequence having one, two or three alterations (preferably amino acid replacements, and more preferably conservative replacements) compared with an amino acid sequence set forth in SEQ ID NO: 11; the VHH CDR3 comprises or consists of an amino acid sequence set forth in SEQ ID NO: 12, or the VHH CDR3 comprises an amino acid sequence having one, two or three alterations (preferably amino acid replacements, and more preferably conservative replacements) compared with an amino acid sequence set forth in SEQ ID NO: 12.

In a preferred embodiment, the VHH in the formula (I) of the antibody molecule disclosed herein that specifically binds to PD-L1 comprises or consists of:
(i) a sequence set forth in SEQ ID NO: 6;
(ii) an amino acid sequence having at least 90%, 91%, 92%, 93%, 94%, 95%, 96%, 97%, 98% or 99% identity to an amino acid sequence set forth in SEQ ID NO: 6; or
(iii) an amino acid sequence having one or more (preferably no more than 10, and more preferably no more than 5, 4, 3, 2, or 1) amino acid alterations (preferably amino acid replacements, and more preferably conservative amino acid replacements) compared with an amino acid sequence set forth in SEQ ID NO: 6, wherein preferably, the amino acid alterations do not occur in the CDRs.

In one embodiment, an antigen-binding site formed by the VH in the formula (I) and the VL in the formula (II) specifically binds to LAG-3, e.g., human LAG-3.

In some specific embodiments, in the antibody molecule disclosed herein, the VH in the formula (I) comprises:
(i) three complementarity determining regions HCDRs of a heavy chain variable region VH set forth in SEQ ID NO: 2, or
(ii) relative to the sequence of (i), a sequence comprising a total of at least one and no more than 5, 4, 3, 2, or 1 amino acid alteration (preferably amino acid replacement, and more preferably conservative replacement) in the three CDRs; and/or
the VL in the formula (II) comprises:
(i) three complementarity determining regions LCDRs of a light chain variable region VL set forth in SEQ ID NO: 8, or
(ii) relative to the sequence of (i), a sequence comprising a total of at least one and no more than 5, 4, 3, 2, or 1 amino acid alteration (preferably amino acid replacement, and more preferably conservative replacement) in the three CDRs.

In some embodiments, in the antibody molecule disclosed herein, the VH in the formula (I) comprises:
complementarity determining regions (CDRs) HCDR1, HCDR2 and HCDR3, wherein the HCDR1 comprises or consists of an amino acid sequence set forth in SEQ ID NO: 13, or the HCDR1 comprises an amino acid sequence having one, two or three alterations (preferably amino acid replacements, and more preferably conservative replacements) compared with an amino acid sequence set forth in SEQ ID NO: 13; the HCDR2 comprises or consists of an amino acid sequence set forth in SEQ ID NO: 14, or the HCDR2 comprises an amino acid sequence having one, two or three alterations (preferably amino acid replacements, and more preferably conservative replacements) compared with an amino acid sequence set forth in SEQ ID NO: 14; the HCDR3 comprises or consists of an amino acid sequence set forth in SEQ ID NO: 15, or the HCDR3 comprises an amino acid sequence having one, two or three alterations (preferably amino acid replacements, and more preferably conservative replacements) compared with an amino acid sequence set forth in SEQ ID NO: 15;
   and/or
the VL in the formula (II) comprises:
   complementarity determining regions (CDRs) LCDR1, LCDR2 and LCDR3, wherein the LCDR1 comprises or consists of an amino acid sequence set forth in SEQ ID NO: 16, or the LCDR1 comprises an amino acid sequence having one, two or three alterations (preferably amino acid replacements, and more preferably conservative replacements) compared with an amino acid sequence set forth in SEQ ID NO: 16; the LCDR2 comprises or consists of an amino acid sequence set forth in SEQ ID NO: 17, or the LCDR2 comprises an amino acid sequence having one, two, or three alterations (preferably amino acid replacements, and more preferably conservative replacements) compared with an amino acid sequence set forth in SEQ ID NO: 17; the LCDR3 comprises or consists of an amino acid sequence set forth in SEQ ID NO: 18, or the LCDR3 comprises an amino acid sequence having one, two or three alterations (preferably amino acid replacements, and more preferably conservative replacements) compared with an amino acid sequence set forth in SEQ ID NO: 18.

In some embodiments, in the antibody molecule disclosed herein,
(a) the VH in the formula (I):
   (i) comprises or consists of an amino acid sequence having at least 90%, 91%, 92%, 93%, 94%, 95%, 96%, 97%, 98% or 99% identity to an amino acid sequence set forth in SEQ ID NO: 2; or
   (ii) comprises or consists of an amino acid sequence set forth in SEQ ID NO: 2; or
   (iii) comprises or consists of an amino acid sequence having one or more (preferably no more than 10, and more preferably no more than 5, 4, 3, 2, or 1) amino acid alterations (preferably amino acid replacements, and more preferably conservative amino acid replacements) compared with an amino acid sequence set forth in SEQ ID NO: 2, wherein preferably, the amino acid alterations do not occur in the CDRs;
   and/or
(b) the VL in the formula (II):
   (i) comprises or consists of an amino acid sequence having at least 90%, 91%, 92%, 93%, 94%, 95%, 96%, 97%, 98% or 99% identity to an amino acid sequence set forth in SEQ ID NO: 8;
   (ii) comprises or consists of an amino acid sequence set forth in SEQ ID NO: 8; or
   (iii) comprises or consists of an amino acid sequence having one or more (preferably no more than 10, and more preferably no more than 5, 4, 3, 2, or 1) amino acid alterations (preferably amino acid replacements, and more preferably conservative amino acid replacements) compared with an amino acid sequence set forth in SEQ ID NO: 8, wherein preferably, the amino acid alterations do not occur in the CDRs.

In some embodiments, the Fc in the formula (I) of the antibody molecule disclosed herein is from IgG1, IgG2 or IgG4. In some embodiments, the Fc is from IgG1. In some embodiments, the Fc:
(i) comprises or consists of an amino acid sequence having at least 85%, 90%, 91%, 92%, 93%, 94%, 95%, 96%, 97%, 98% or 99% identity to an amino acid sequence set forth in SEQ ID NO: 4;
(ii) comprises or consists of an amino acid sequence set forth in SEQ ID NO: 4; or
(iii) comprises or consists of an amino acid sequence having one or more (preferably no more than 10, and more preferably no more than 5, 4, 3, 2, or 1) amino acid alterations (preferably amino acid replacements, and more preferably conservative amino acid replacements) compared with an amino acid sequence set forth in SEQ ID NO: 4.

In some embodiments, the CHI in the formula (I) of the antibody molecule disclosed herein is from IgG1, IgG2 or IgG4. In some embodiments, the CHI is from IgG1. In some embodiments, the CH1:
(i) comprises or consists of an amino acid sequence having at least 90%, 91%, 92%, 93%, 94%, 95%, 96%, 97%, 98% or 99% identity to an amino acid sequence set forth in SEQ ID NO: 3;
(ii) comprises or consists of an amino acid sequence set forth in SEQ ID NO: 3; or
(iii) comprises or consists of an amino acid sequence having one or more (preferably no more than 10, and more preferably no more than 5, 4, 3, 2, or 1) amino acid alterations (preferably amino acid replacements, and more preferably conservative amino acid replacements) compared with an amino acid sequence set forth in SEQ ID NO: 3.

In some embodiments, the CL in the formula (I) of the antibody molecule disclosed herein:
(i) comprises or consists of an amino acid sequence having at least 90%, 91%, 92%, 93%, 94%, 95%, 96%, 97%, 98% or 99% identity to an amino acid sequence set forth in SEQ ID NO: 9;
(ii) comprises or consists of an amino acid sequence set forth in SEQ ID NO: 9; or
(iii) comprises or consists of an amino acid sequence having one or more (preferably no more than 10, and more preferably no more than 5, 4, 3, 2, or 1) amino acid alterations (preferably amino acid replacements, and more preferably conservative amino acid replacements) compared with an amino acid sequence set forth in SEQ ID NO: 9.

In some embodiments, the X in the formula (I) of the antibody molecule disclosed herein is a flexible linker, e.g., a linker having glycine and/or serine residues present alone or in combination. In one embodiment, the linker comprises an amino acid sequence (Gly₄Ser)n, wherein n is a positive integer equal to or greater than 1, e.g., n is a positive integer from 1 to 7, such as 2, 3, 4, 5 or 6. In one embodiment, n is 1, 2, 3 or 4. In one embodiment, the X has a sequence set forth in SEQ ID NO: 5.

In some embodiments, in the antibody molecule disclosed herein,
(a) the VH-CH1-Fc in the formula (I):
   (i) comprises or consists of an amino acid sequence having at least 85%, 90%, 91%, 92%, 93%, 94%, 95%, 96%, 97%, 98% or 99% identity to an amino acid sequence set forth in SEQ ID NO: 19;
   (ii) comprises or consists of an amino acid sequence set forth in SEQ ID NO: 19; or
   (iii) comprises an amino acid sequence having one or more (preferably no more than 20 or 10, and more preferably no more than 5, 4, 3, 2, or 1) amino acid alterations (preferably amino acid replacements, and more preferably conservative amino acid replacements) compared with an amino acid sequence set forth in SEQ ID NO: 19, wherein preferably, the amino acid alterations do not occur in the CDRs of the heavy chain, and more preferably, the amino acid alterations do not occur in the heavy chain variable region;
   and/or
(b) the VL-CL of the formula (II):
   (i) comprises or consists of an amino acid sequence having at least 85%, 90%, 91%, 92%, 93%, 94%, 95%, 96%, 97%, 98% or 99% identity to an amino acid sequence set forth in SEQ ID NO: 7;
   (ii) comprises or consists of an amino acid sequence set forth in SEQ ID NO: 7; or
   (iii) comprises an amino acid sequence having one or more (preferably no more than 20 or 10, and more preferably no more than 5, 4, 3, 2, or 1) amino acid alterations (preferably amino acid replacements, and more preferably conservative amino acid replacements) compared with an amino acid sequence set forth in SEQ ID NO: 7, wherein preferably, the amino acid alterations do not occur in the CDRs of the light chain, and more preferably, the amino acid alterations do not occur in the light chain variable region.

In some embodiments, the antibody molecule disclosed herein further comprises a signal peptide sequence, e.g., METDTLLLWVLLLWVPGSTG (SEQ ID NO: 20), at the N-terminus of the VH in the formula (I) or the VL in the formula (II).

In a preferred embodiment of the present invention, the present invention relates to an antibody molecule in which the polypeptide chain of formula (I) comprises a sequence set forth in SEQ ID NO: 1 or an amino acid sequence having at least 85%, 90%, 91%, 92%, 93%, 94%, 95%, 96%, 97%, 98% or 99% identity thereto, and/or the polypeptide chain of formula (II) comprises a sequence set forth in SEQ ID NO: 7 or an amino acid sequence having at least 85%, 90%, 91%, 92%, 93%, 94%, 95%, 96%, 97%, 98% or 99% identity thereto.

In one embodiment, the antibody disclosed herein comprises a whole antibody portion as a portion thereof. In some embodiments, the whole antibody portion disclosed herein comprises the VH-CH1-Fc of the chain of formula (I) and the VL-CL of the chain of formula (II), wherein the VH constitutes the heavy chain variable region of the whole antibody portion, the CH1-Fc constitutes the heavy chain constant region of the whole antibody portion, and the two together constitute the heavy chain of the whole antibody portion; the VL constitutes the light chain variable region of the whole antibody portion, the CL constitutes the light chain constant region of the whole antibody portion, and the two together constitute the light chain of the whole antibody portion.

In some embodiments, the whole antibody portion is a human antibody. In some embodiments, the whole antibody portion is an antibody in the form of IgG1, an antibody in the form of IgG2 or an antibody in the form of IgG4. In some embodiments, the whole antibody portion may independently constitutes a monoclonal antibody. In some embodiments, the whole antibody portion is humanized. In some embodiments, the whole antibody portion is a chimeric antibody. In some embodiments, at least a portion of a framework sequence of the whole antibody portion is a human consensus framework sequence.

In a specific embodiment, the whole antibody portion is an anti-LAG-3 antibody.

In one embodiment of the present invention, the amino acid alteration described herein includes amino acid replacement, insertion or deletion. Preferably, the amino acid alteration described herein is an amino acid replacement, preferably a conservative replacement.

In a preferred embodiment, the amino acid alteration described herein occurs in a region outside the CDR (e.g., in FR). More preferably, the amino acid alteration described herein occurs in a region outside the heavy chain variable region and/or outside the light chain variable region.

In some embodiments, the replacement is a conservative replacement. A conservative replacement refers to the replacement of an amino acid by another amino acid of the same class, e.g., the replacement of an acidic amino acid by another acidic amino acid, the replacement of a basic amino acid by another basic amino acid, or the replacement of a neutral amino acid by another neutral amino acid. Exemplary replacements are shown in Table A below:

**Table A**

| Original residues | Exemplary replacement | Preferred replacement |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

In certain embodiments, the antibody provided herein is altered to increase or decrease the extent to which the antibody is glycosylated. Addition or deletion of glycosylation sites of an antibody can be conveniently achieved by altering the amino acid sequence to create or remove one or more glycosylation sites.

For example, one or more amino acid replacements can be performed to eliminate one or more variable region framework glycosylation sites, thereby eliminating glycosylation at the site(s). Such aglycosylation can increase the affinity of an antibody to an antigen. See, e.g., US Patent Nos. 5,714,350 and 6,350,861. Antibodies with altered classes of glycosylation can be prepared, such as low-fucosylated antibodies with reduced amounts of fucosyl residues or antibodies with increased bisecting GlcNac structures. Such altered glycosylation patterns have shown the ability to increase ADCC of antibodies. Such carbohydrate modifications can be achieved by, e.g., expressing antibodies in host cells with altered glycosylation systems. Alternatively, fucosidase can be used to cut fucosyl residues off the antibody; for example, α-L-fucosidase removes fucosyl residues from the antibody (Tarentino et al. (1975), Biochem., 14:5516-23).

In certain embodiments, one or more amino acid modifications can be introduced into the Fc region of the antibody provided herein, thereby producing an Fc region variant to, for example, enhance the efficacy of the antibody in treating cancer or a cell proliferative disease. Modifications of the Fc region include amino acid alteration (replacement, deletion and insertion), glycosylation or deglycosylation, and addition of multiple Fc. Modifications to Fc can also alter the antibody half-life of the therapeutic antibodies, thereby enabling less frequent administrations, and the resulting increased convenience and reduced material use. See Presta (2005), J. Allergy Clin. Immunol. 116:731, pp. 734-735.

In one embodiment, the number of cysteine residues of an antibody can be changed to modify antibody properties. For example, the hinge region of CHI is modified to change (e.g., increase or decrease) the number of cysteine residues in the hinge region. This method is further described in US Patent No. 5,677,425. The number of cysteine residues in the hinge region of CHI can be changed to, e.g., facilitate assembly of the light and heavy chains, or increase or decrease the stability of the antibody.

Optionally, the antibody disclosed herein comprises post-translational modifications to the antibody chain. Exemplary post-translational modifications include disulfide bond formation, glycosylation, lipidation, acetylation, phosphorylation, or any other operations, such as conjugation with a labeling component.

In one embodiment of the present invention, the antibody or the fragment described herein is glycosylated with an engineered yeast N-linked glycan or CHO N-linked glycan.

In certain embodiments, the antibody provided herein can be further modified to comprise other non-protein portions known in the art and readily available. Suitable portions for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

Another modification, encompassed by the present invention, to the antibody or the fragment thereof described herein is PEGylation. An antibody can be PEGylated to, for example, increase the biological (e.g., serum) half-life of the antibody. As used herein, the term "polyethylene glycol" is intended to encompass any form of PEG which has been used to derivatize other proteins, such as mono (C₁-C₁₀)alkoxy- or aryloxy polyethylene glycol or polyethylene glycol-maleimide. In certain embodiments, the antibody to be PEGylated is an aglycosylated antibody. Methods for PEGylation of proteins are known in the art (e.g., see EP 0154316 and EP 0401384), and can be applied to the antibody disclosed herein.

In some embodiments, the antibody molecule disclosed herein is humanized. Different methods for humanizing antibodies are known to those skilled, as summarized in an article by Almagro & Fransson, the content of which is incorporated herein by reference in its entirety (Almagro J.C. and Fransson J (2008), Frontiers in Bioscience 13:1619-1633).

In some embodiments, the antibody molecule disclosed herein is a human antibody or a humanized antibody. The human or humanized antibody can be prepared using a variety of techniques known in the art.

In some embodiments, the antibody molecule disclosed herein is a chimeric antibody.

In some embodiments, at least a portion of the framework sequences of the antibody molecule disclosed herein is a human consensus framework sequence.

In one embodiment, the antibody molecule disclosed herein also encompasses an antibody fragment thereof, such as Fab, Fab', Fab'-SH, Fv, single-chain antibody (e.g., scFv) or (Fab')₂, or linear antibody.

In some embodiments, the bispecific antibody disclosed herein has one or more of the following properties:
(1) the bispecific antibody or the fragment thereof disclosed herein simultaneously binds to two antigens with high affinity, e.g., binds to LAG-3 (e.g., human LAG-3) with an equilibrium dissociation constant (K_{D}) of less than or equal to about 10 nM, 9 nM, 8 nM, 7 nM, 6 nM, 5 nM, 4 nM, 3 nM or 2 nM (most preferably, the K_{D} is less than or equal to about 1.9 nM or 1.8 nM, and in some embodiments, the K_{D} is about 1 nM, 1.1 nM, 1.2 nM, 1.3 nM, 1.4 nM, 1.5 nM, 1.6 nM or 1.7 nM or more), and simultaneously, binds to PD-L1 (e.g., human PD-L1) with an equilibrium dissociation constant (K_{D}) of less than or equal to about 100 nM, 90 nM, 80 nM, 70 nM, 60 nM, 50 nM, 40 nM, 30 nM or 20 nM (most preferably, the K_{D} is less than or equal to about 19 nM, 18 nM or 17 nM, and in some embodiments, the K_{D} is about 10 nM, 11 nM, 12 nM, 13 nM, 14 nM, 15 nM or 16 nM or more); in some embodiments, the antibody binding affinity is determined using biological optical interferometry (e.g., Fortebio affinity assay).
(2) the antibody or the fragment thereof disclosed herein binds to cells expressing human PD-L1, e.g., with an EC₅₀ of less than or equal to about 3 nM, 2 nM, 1.5 nM, 1.4 nM, 1.3 nM, 1.2 nM or 1.1 nM (in some embodiments, the EC₅₀ is about 0.5 nM, 0.6 nM, 0.7 nM, 0.8 nM, 0.9 nM or 1.0 nM or more), and simultaneously, binds to cells expressing human LAG-3, e.g., with an EC₅₀ of less than or equal to about 3 nM, 2 nM, 1.5 nM, 1.4 nM, 1.3 nM, 1.2 nM, 1.1 nM, 1 nM, 0.9 nM, 0.8 nM, 0.7 nM, 0.6 nM or 0.5 nM (in some embodiments, the EC₅₀ is about 0.3 nM or 0.4 nM or more). In some embodiments, the binding is determined using flow cytometry (e.g., FACS). In some embodiments, the cells expressing human LAG-3 are 293 cells (e.g., HEK293 cells) expressing human LAG-3, and/or the cells expressing human PD-L1 are CHO cells (e.g., CHOS cells) expressing human PD-L1.
(3) the antibody or the fragment thereof disclosed herein has good thermal stability. In some embodiments, the antibody or the fragment thereof has a Tm of greater than or equal to about 60 °C, 61 °C, 62 °C or 63 °C as determined by differential scanning fluorimetry.
(4) The antibody or the fragment thereof disclosed herein activates CD4+ T cells (e.g., human CD4+ T cells), e.g., promotes expression of IL-2 thereof.
(5) the antibody or the fragment thereof disclosed herein inhibits one or more activities of LAG-3 and PD-L1, e.g., resulting in one or more of the following: blocking of the binding of PD-1 to PD-L1 or PD-L2 to PD-1, increased antigen-dependent stimulation of CD4+ T lymphocytes; increased T cell proliferation; increased expression of activating antigens (e.g., CD25); increased expression of cytokines (e.g., interferon-y (IFN-γ), interleukin-2 (IL-2), or interleukin-4 (IL-4)); increased expression of chemokines (e.g., CCL3, CCL4, or CCL5); decreased suppressive activity of Treg cells; increased T-cell homeostasis; increased tumor-infiltrating lymphocytes; or reduced immune evasion of cancer cells.
(6) the antibody or the fragment thereof disclosed herein may induce antibody-dependent cell-mediated cytotoxicity (ADCC).

In some embodiments, the antibody or the antigen-binding fragment thereof disclosed herein has one or more of the following characteristics:
(i) showing the same or similar binding affinity and/or specificity for LAG-3 and PD-L1 as the antibody disclosed herein (e.g., comprising SEQ ID NO: 1 as the peptide chain of the formula (I) and SEQ ID NO: 7 as the peptide chain of the formula (II));
(ii) inhibiting (e.g., competitively inhibiting) the binding of the antibody disclosed herein (e.g., comprising SEQ ID NO: 1 as the peptide chain of the formula (I) and SEQ ID NO: 7 as the peptide chain of the formula (II)) to LAG-3 and PD-L1;
(iii) binding to the same or overlapping epitope as the antibody disclosed herein (e.g., comprising SEQ ID NO: 1 as the peptide chain of the formula (I) and SEQ ID NO: 7 as the peptide chain of the formula (II));
(iv) competing with the antibody disclosed herein (e.g., comprising SEQ ID NO: 1 as the peptide chain of the formula (I) and SEQ ID NO: 7 as the peptide chain of the formula (II)) for binding to LAG-3 and PD-L1; and
(v) having one or more biological properties of the antibody disclosed herein (e.g., comprising SEQ ID NO: 1 as the peptide chain of the formula (I) and SEQ ID NO: 7 as the peptide chain of the formula (II)).

### III. Immunoconjugate

In some embodiments, the present invention further provides an antibody ("an immunoconjugate") conjugated to additional substances. In some embodiments, the additional substance is, for example, a therapeutic agent or a label, such as a cytotoxic agent, an immunosuppressive or a chemotherapeutic agent. The cytotoxic agent includes any agent that is harmful to cells. Examples of the cytotoxic agent (e.g., chemotherapeutic agent) suitable for forming the immunoconjugate are known in the art.

In addition, the antibody molecule disclosed herein can be conjugated to a labeled sequence (such as a peptide) to facilitate purification. In a preferred embodiment, the labeled amino acid sequence is a hexahistidine peptide, such as tags provided in a pQE vector (QIAGEN, Inc., 9259 Eton Avenue, Chatsworth, CA, 91311), etc., many of which are commercially available. As described in Gentz et al., 1989, Proc. Natl. Acad. Sci. USA 86: 821-824, for example, a hexahistidine provides convenient purification of fusion proteins. Other peptide tags for purification include, but are not limited to, hemagglutinin ("HA") tags, which correspond to epitopes derived from influenza hemagglutinin proteins (Wilson et al., 1984, Cell 37: 767), and "flag" tags.

In other embodiments, the antibody molecule disclosed herein is conjugated to a diagnostic or detectable agent. Such antibodies can be used as a part of clinical test methods (e.g., to determine the effect of a particular therapy), for monitoring or predicting the onset, development, progression, and/or severity of a disease or a condition. Such diagnosis and detection can be achieved by coupling the antibody to a detectable substance, which includes, but is not limited to, a variety of enzymes, prosthetic groups, fluorescent substances, luminescent substances, and positron-emitting metal and non-radioactive paramagnetic metal ions used in various positron emission imaging techniques.

In addition, the antibody molecule disclosed herein can be conjugated to a therapeutic or drug moiety that modulates a given biological response. The therapeutic or drug moiety should not be interpreted as limited to classic chemotherapeutics. For example, the drug moiety may be a protein, a peptide or a polypeptide possessing the desired biological activity. Such proteins may, for example, include toxins, proteins or biological response modifiers.

In addition, the antibody molecule disclosed herein can be conjugated to a therapeutic moiety such as a radioactive metal ion, or a macrocyclic chelating agent that may be used to conjugate radioactive metal ions to polypeptides. Techniques for conjugating a therapeutic moiety to an antibody are well known, see, e.g., Arnon et al., Monoclonal Antibodies For Immunotargeting Of Drugs In Cancer Therapy, cited in Monoclonal Antibodies And Cancer Therapy, Reisfeld et al. (authoring), pp. 243-256 (Alan R. Liss, Inc. 1985).

Antibodies can also be attached to a solid phase support, which is particularly useful for immunoassays or purification of target antigens. Such solid phase supports include, but are not limited to, glass, cellulose, polyacrylamide, nylon, polystyrene, polyvinyl chloride, or polypropylene.

In some embodiments, the immunoconjugate is used to prevent or treat a disease, such as an autoimmune disease, an inflammatory disease, an infection or a tumor. For example, the disease is a tumor (e.g., cancer) or an infection. In some embodiments, the tumor is a tumor immune escape. Preferably, the tumor is, for example, colon cancer or colorectal cancer or rectal cancer.

### IV. Nucleic acid disclosed herein and host cell comprising same

In one aspect, the present invention provides a nucleic acid encoding any of the above antibodies or the fragments thereof or any one of the chains thereof. In one embodiment, provided is a vector comprising the nucleic acid. In one embodiment, the vector is an expression vector. In one embodiment, provided is a host cell comprising the nucleic acid or the vector. In one embodiment, the host cell is eukaryotic. In another embodiment, the host cell is selected from a yeast cell, a mammal cell (e.g., CHO cell or 293 cell), and other cells suitable for preparing an antibody or an antigen-binding fragment thereof. In another embodiment, the host cell is prokaryotic.

For example, the nucleic acid disclosed herein includes a nucleic acid encoding an amino acid sequence selected from any one of SEQ ID NOs: 1-9, or a nucleic acid encoding an amino acid sequence having at least 85%, 90%, 91%, 92%, 93%, 94%, 95%, 96%, 97%, 98% or 99% identity to an amino acid sequence selected from any one of SEQ ID NOs: 1-9.

The present invention further provides a nucleic acid that is hybridized under a stringent condition with the following nucleic acid or a nucleic acid having one or more replacements (e.g., conservative replacements), deletions or insertions compared with the following nucleic acid: a nucleic acid encoding an amino acid sequence selected from any one of SEQ ID NOs: 1-9; or a nucleic acid encoding an amino acid sequence having at least 85%, 90%, 91%, 92%, 93%, 94%, 95%, 96%, 97%, 98% or 99% identity to an amino acid sequence selected from any one of SEQ ID NOs: 1-9.

In one embodiment, provided are one or more vectors comprising the nucleic acid. In one embodiment, the vector is an expression vector, such as a eukaryotic expression vector. The vector includes, but is not limited to, a virus, a plasmid, a cosmid, a λ phage, or a yeast artificial chromosome (YAC). In some embodiments, the vector is a pTT5 vector.

Once the expression vector or DNA sequence has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, lipid-based transfection, or other conventional techniques. In the case of protoplast fusion, cells are incubated in a culture medium and screened for appropriate activity. Methods and conditions for incubating the resulting transfected cells and for isolating the resulting antibody molecules are known to those skilled in the art and may be varied or optimized according to the particular expression vector and the particular mammalian host cell used based on the present description and methods known in the art.

Additionally, cells having stably incorporated DNA in chromosomes thereof can be selected by introducing one or more markers permitting the selection of transfected host cells. The markers may, for example, provide prototrophy, biocidal (e.g., antibiotics) resistance, or heavy metal (e.g., copper) resistance, etc., for an auxotrophic host. Selectable marker genes may be connected directly to a DNA sequence to be expressed or introduced through co-transformation into the same cell. Additional elements may also be required for optimal synthesis of mRNA. The elements may include splicing signals, transcriptional promoters, enhancers, and termination signals.

In one embodiment, provided is a host cell comprising one or more polynucleotides disclosed herein. In some embodiments, provided is a host cell comprising the expression vector disclosed herein. In some embodiments, the host cell is selected from a yeast cell, a mammalian cell, or other cells suitable for preparing an antibody or an antigen-binding fragment thereof.

Suitable host cells comprise prokaryotic microorganisms, such as *E.coli.* The host cells may also be eukaryotic microorganisms such as filamentous fungi or yeast, or various eukaryotic cells such as insect cells. Vertebrate cells may also be used as hosts. For example, a mammalian cell line engineered to be suitable for suspension growth may be used. Examples of useful mammalian host cell lines include monkey kidney CV1 line (COS-7) transformed by SV40; human embryonic kidney line (HEK 293 or 293F cells), 293 cell, baby hamster kidney cell (BHK), monkey kidney cell (CV1), African green monkey kidney cell (VERO-76), human cervical cancer cell (HELA), canine kidney cell (MDCK), buffalo rat liver cell (BRL 3A), human lung cell (W138), human liver cell (Hep G2), Chinese hamster ovary cell (CHO cell), CHOS cell, NSO cell, and myeloma cell line such as Y0, NS0, P3X63 and Sp2/0. For reviews of mammalian host cell lines suitable for protein production, see, e.g., Yazaki and Wu, Methods in Molecular Biology, vol. 248 (edited by B. K. C. Lo, Humana Press, Totowa, NJ), pp. 255-268 (2003). In a preferred embodiment, the host cell is a CHO cell (e.g., a CHOS cell) or a 293 cell (e.g., a HEK293 cell).

### V. Production and purification of the antibody molecule disclosed herein

In one embodiment, the present invention provides a method for preparing the antibody molecule or the fragment (preferably the antigen-binding fragment) thereof disclosed herein, wherein the method comprises culturing the host cell under conditions suitable for expressing the nucleic acid encoding the antibody molecule or the fragment (preferably the antigen-binding fragment) thereof disclosed herein, and optionally isolating the antibody or the fragment (preferably the antigen-binding fragment) thereof. In a certain embodiment, the method further comprises isolating the antibody molecule or the fragment (preferably the antigen-binding fragment) thereof disclosed herein from the host cell.

In one embodiment, provided is a method for preparing the antibody molecule disclosed herein, wherein the method comprises culturing the host cell comprising a nucleic acid encoding the antibody (e.g., any one and/or more polypeptide chains) or an expression vector comprising the nucleic acid, as provided above, under conditions suitable for expressing antibodies, and optionally isolating the antibody from the host cell (or the host cell medium).

For recombinant production of the antibody molecule disclosed herein, a nucleic acid encoding the antibody (e.g., the antibody described above, e.g., any one and/or more polypeptide chains) is isolated and inserted into one or more vectors for further cloning and/or expressing in the host cells. Such a nucleic acid can be easily isolated and sequenced by using conventional procedures (e.g., by using oligonucleotide probes that are capable of specifically binding to genes encoding heavy and light chains of antibodies).

The antibody molecule prepared as described herein can be purified by known prior art such as high performance liquid chromatography, ion exchange chromatography, gel electrophoresis, affinity chromatography, and size exclusion chromatography. The actual conditions used to purify a particular protein also depend on factors such as net charge, hydrophobicity and hydrophilicity, and these will be apparent to those skilled in the art. The purity of the antibody molecule disclosed herein can be determined by any one of a variety of well-known analytical methods including size exclusion chromatography, gel electrophoresis, high performance liquid chromatography, and the like.

### VI. Assays

The antibody molecule provided herein can be identified, screened, or characterized for physical/chemical properties and/or biological activities thereof through a variety of assays known in the art. In one aspect, the antibody disclosed herein is tested for the antigen-binding activity, for example, by known methods such as ELISA and Western blotting. The binding of the antibody to an antigen can be determined by methods known in the art, and exemplary methods are disclosed herein, such as bio-layer interferometry.

The present invention also provides an assay for identifying antibodies having biological activities. Biological activities may include, for example, binding to an antigen, binding to an antigen on the cell surface, and inhibition of an antigen. Further provided is an antibody having such biological activities *in vivo* and/or *in vitro.*

In certain embodiments, the antibody disclosed herein is characterized for such biological activities.

The present invention also provides methods for identifying properties of an antibody, such as druggability-related properties. The druggability-related properties include, for example, thermal stability.

Cells for use in any of the *in vitro* assays described above include cell lines that naturally express an antigen or are engineered to express the antigen. Such cells also include cell lines that express the antigen and cell lines that do not normally express the antigen but have been transfected with a DNA encoding the antigen.

It will be understood that any of the above assays can be performed by using the immunoconjugate disclosed herein in place of or in addition to the antibody molecule disclosed herein.

It will also be understood that any of the above assays can be performed on the antibody molecule disclosed herein and other active agents.

In some embodiments, the antigen is PD-L1 (e.g., human PD-L1) and/or LAG-3 (e.g., human LAG-3).

### VII. Pharmaceutical composition and pharmaceutical preparation

In some embodiments, the present invention provides a composition comprising any of the antibody molecules or the fragments (preferably the antigen-binding fragments) thereof described herein, or the immunoconjugates thereof, wherein, preferably, the composition is a pharmaceutical composition. In one embodiment, the composition further comprises a pharmaceutical supplementary material. In one embodiment, the composition, e.g., the pharmaceutical composition, comprises the antibody molecule or the fragment thereof disclosed herein or the immunoconjugate thereof, and a combination of one or more additional therapeutic agents (e.g., chemotherapeutic agents, cytotoxic agents, vaccines, additional antibodies, anti-infective active agents, small molecule drugs, or immunomodulatory agents).

In some embodiments, the composition is used for preventing or treating a disease, such as an autoimmune disease, an inflammatory disease, an infection or a tumor. For example, the disease is a tumor (e.g., cancer) or an infection. In some embodiments, the tumor is a tumor immune escape. Preferably, the tumor is, for example, colon cancer or colorectal cancer or rectal cancer.

The present invention also includes a composition (including a pharmaceutical composition or a pharmaceutical preparation) comprising the antibody disclosed herein or the immunoconjugate thereof, and/or a composition (including a pharmaceutical composition or a pharmaceutical preparation) comprising a polynucleotide encoding the antibody disclosed herein. In certain embodiments, the composition comprises one or more of the antibodies or the fragments thereof disclosed herein, or one or more polynucleotides encoding one or more of the antibodies or the fragments thereof disclosed herein.

Such compositions can further comprise a suitable pharmaceutical supplementary material, such as a pharmaceutical carrier or a pharmaceutical excipient known in the art, including buffers.

As used herein, the "pharmaceutical carrier" includes any and all solvents, dispersion media, isotonic agents and absorption delaying agents, and the like that are physiologically compatible. The pharmaceutical carrier suitable for use in the present invention can be sterile liquid, such as water and oil, including petroleum, or oil of an animal, vegetable or a synthetic source, e.g., peanut oil, soybean oil, mineral oil, and sesame oil. Water is a preferred carrier when the pharmaceutical composition is administered intravenously. Saline solutions, aqueous dextrose and glycerol solutions can also be employed as liquid carriers, particularly for injectable solutions.

Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. For use and application of excipients, see Handbook of Pharmaceutical Excipients, 5th Ed., R. C. Rowe, P. J. Seskey and S. C. Owen, Pharmaceutical Press, London, Chicago.

The composition may further comprise a small quantity of wetting agent, emulsifier, or pH buffer, if desired. The compositions may be in the form of a solution, a suspension, an emulsion, a tablet, a pill, a capsule, a powder, a sustained release preparation, and the like. Oral preparations may comprise standard pharmaceutical carriers and/or excipients such as pharmaceutical-grade mannitol, lactose, starch, magnesium stearate and saccharin.

The compositions disclosed herein may be in a variety of forms. These forms include, for example, liquid, semi-solid and solid dosage forms, such as liquid solutions (e.g., injectable solutions and infusible solutions), dispersions or suspensions, liposomes, and suppositories.

The preferred form depends on the intended mode of administration and therapeutic use. Commonly preferred compositions are in the form of injectable solutions or infusible solutions. The preferred mode of administration is parenteral (e.g., intravenous, subcutaneous, intraperitoneal (i.p.) and intramuscular) injection. In one preferred embodiment, the antibody molecule is administered by intravenous infusion or injection. In another preferred embodiment, the antibody molecule is administered by intramuscular, intraperitoneal or subcutaneous injection.

The pharmaceutical preparation, preferably in the form of a lyophilized preparation or an aqueous solution, comprising the antibody described herein can be prepared by mixing the antibody of desired purity with one or more optional pharmaceutical supplementary materials (Remington's Pharmaceutical Sciences, 16th Ed., Osol, A. ed. (1980)).

An exemplary lyophilized antibody preparation is described in U.S. Patent No. 6,267,958. The aqueous antibody preparation includes those described in U.S. Patent No. 6,171,586 and WO2006/044908, and the latter preparation comprises a histidine-acetate buffer.

The pharmaceutical composition or preparation disclosed herein may also comprise more than one active ingredient required by a particular indication treated, preferably those having complementarity activities without adversely affecting one another. For example, it may be desirable to also provide additional therapeutic agents, such as chemotherapeutic agents, cytotoxic agents, vaccines, additional antibodies, anti-infective active agents, small molecule drugs, or immunomodulatory agents. The active ingredients are suitably combined in an amount effective for an intended purpose.

In some embodiments, the therapeutic agent is selected from one, two or all of the following categories (i)-(iii): (i) drugs that enhance antigen presentation (e.g., tumor antigen presentation); (ii) drugs that enhance effector cell responses (e.g., B cell and/or T cell activation and/or mobilization); or (iii) drugs that reduce immunosuppression.

A sustained release preparation can be prepared. Suitable examples of the sustained release preparation include a semipermeable matrix of a solid hydrophobic polymer containing an antibody. The matrix is in the form of a shaped article, such as a film or a microcapsule.

The pharmaceutical composition disclosed herein is suitable for intravenous, intramuscular, subcutaneous, parenteral, rectal, spinal or epidermal administration (e.g., by injection or infusion).

Therapeutic compositions generally should be sterile and stable under the conditions of manufacture and storage. The compositions can be prepared into solutions, microemulsions, dispersions, liposomes, or lyophilized forms. Sterile injectable solutions can be prepared by adding a required amount of an active compound (i.e., antibody molecule) to a suitable solvent, and then filtering and disinfecting the resulting mixture. Generally, dispersions are prepared by incorporating the active compound into a sterile vehicle, which comprises a basic dispersion medium and other ingredients. Coating agents such as lecithin can be used. In the case of dispersions, the proper fluidity of a solution can be maintained by using a surfactant. Prolonged absorption of injectable compositions can be caused by including in the compositions a substance that delays absorption such as monostearate and gelatin.

A kit comprising the antibody molecule described herein is also within the scope of the present invention. The kit may include one or more other elements, including, for example, package insert; other reagents, such as a marker or a reagent for coupling; a pharmaceutical carrier; and a device or other materials for administration to a subject.

### VIII. Combination product or kit

In some embodiments, the present invention also provides a combination product comprising the antibody or the antigen-binding fragment thereof disclosed herein, or the immunoconjugate thereof, and one or more additional therapeutic agents (e.g., chemotherapeutic agents, additional antibodies, cytotoxic agents, vaccines, anti-infective active agents, small molecule drugs or immunomodulatory agents). In some embodiments, the composition product is used for preventing or treating a disease, such as an autoimmune disease, an inflammatory disease, an infection or a tumor. For example, the disease is a tumor (e.g., cancer) or an infection. In some embodiments, the tumor is a tumor immune escape. Preferably, the tumor is, for example, colon cancer or colorectal cancer or rectal cancer. In some embodiments, two or more of the ingredients in the combination product may be administered to a subject in combination sequentially, separately or simultaneously.

In some embodiments, the present invention also provides a kit comprising the antibody, the pharmaceutical composition, the immunoconjugate or the combination product disclosed herein, and optionally a package insert directing administration.

In some embodiments, the present invention also provides a pharmaceutical product comprising the antibody, the pharmaceutical composition, the immunoconjugate or the combination product disclosed herein, and optionally a package insert directing administration.

### IX. Use of the antibody molecule disclosed herein

In one aspect, the present invention relates to use of the antibody molecule disclosed herein *in vivo* in treating or preventing diseases requiring modulation of an immune response in a subject, thereby inhibiting or reducing the occurrence or recurrence of related diseases such as a cancerous tumor, an autoimmune disease and an infectious disease. The antibody molecule disclosed herein may be used alone. Alternatively, the antibody molecule may be administered in combination with other therapies (e.g., a therapeutic agent, a prophylactic agent or a therapeutic modality). When the antibody molecule disclosed herein is administered in combination with one or more other drugs, such combinations can be administered sequentially in any order, separately or simultaneously.

Accordingly, in one embodiment, the present invention provides a method for modulating an immune response in a subject, wherein the method comprises administering to the subject a therapeutically effective amount of the antibody molecule described herein. In another embodiment, the present invention provides a method for preventing the occurrence or recurrence of a disease in a subject, wherein the method comprises administering to the subject a prophylactically effective amount of the antibody molecule described herein.

In some embodiments, the disease is one in a patient that has (e.g., an elevated level of, e.g., an elevated protein or nucleic acid level of) LAG-3. In some embodiments, the disease is one in a patient that has (e.g., an elevated level of, e.g., an elevated protein or nucleic acid level of) PD-L1, PD-1 or PD-L2. In some embodiments, the disease is one in a patient that has (e.g., elevated levels of, e.g., elevated protein or nucleic acid levels of) LAG-3 and PD-L1, PD-1 or PD-L2, e.g., cancer. In some embodiments, the disease is one that would benefit from inhibition of nucleic acid or protein levels of LAG-3 and PD-L1, PD-1 or PD-L2. In some embodiments, the disease benefits from blocking the binding of PD-1 to PD-L1, or the binding of PD-1 to PD-L2.

In some embodiments, the present invention relates to a method for inhibiting antigenic action in an individual, wherein the method comprises administering to a subject an effective amount of the antibody molecule (e.g., the anti-PD-L1/LAG3 antibody), the pharmaceutical composition, the immunoconjugate, the combination product or the kit disclosed herein. In some embodiments, the antigen is LAG-3 (e.g., human LAG-3) and/or PD-L1 (e.g., human PD-L1). In some embodiments, inhibiting antigenic action refers to blocking the binding of PD-1 to PD-L1, or blocking the binding of PD-1 to PD-L2, and/or inhibiting action of LAG-3.

In another aspect, the present invention relates to a method for preventing or treating a tumor (e.g., cancer) in a subject, wherein the method comprises administering to the subject an effective amount of the antibody molecule, the pharmaceutical composition, the immunoconjugate, the combination product or the kit disclosed herein. In some embodiments, the tumor is a tumor immune escape. In some embodiments, the tumor is cancer.

In another aspect, the present invention relates to a method for preventing or treating an infectious disease in a subject, wherein the method comprises administering to the subject an effective amount of the antibody molecule, the pharmaceutical composition, the immunoconjugate, the combination product or the kit disclosed herein. In another aspect, the present invention relates to a method for preventing or treating an autoimmune disease and/or an inflammatory disease in a subject, wherein the method comprises administering to the subject an effective amount of the antibody molecule, the pharmaceutical composition, the immunoconjugate, the combination product or the kit disclosed herein.

The subject may be a mammal, e.g., a primate, preferably a higher primate, e.g., a human (e.g., a patient suffering from or at risk of suffering from the disease described herein). In one embodiment, the subject suffers from or is at risk of suffering from the disease described herein (e.g., the tumor, infection or autoimmune disease described herein). In certain embodiments, the subject is receiving or has received additional therapies, e.g., chemotherapy and/or radiotherapy. Alternatively or in combination, the subject is immunocompromised due to infection or is at risk of being immunocompromised due to infection.

In some embodiments, cancers treated and/or prevented with the antibody molecule include, but are not limited to, solid tumors, hematological cancers and metastatic lesions. In one embodiment, the cancer is a solid tumor. Examples of solid tumors include malignant tumors. The cancer may be at an early, intermediate or advanced stage, or may be a metastatic cancer.

The methods and compositions disclosed herein may be used to treat metastatic lesions associated with the aforementioned cancers.

In some embodiments, the tumor is one that expresses (e.g., an elevated level of) LAG-3, such as cancer. In some embodiments, the tumor is one that expresses (e.g., an elevated level of) PD-L1, such as cancer. In some embodiments, the tumor is one that expresses (e.g., elevated levels of) LAG-3 and PD-L1, such as cancer.

In some embodiments, infectious diseases treated and/or prevented with the antibody molecule include pathogens for which no effective vaccine is currently available or pathogens for which conventional vaccines have not been fully effective. The blocking effect of the antibody molecule exemplified by the present invention on PD-L1 can be particularly used to fight infections caused by pathogens that develop mutant antigens as the infection progresses. These mutant antigens can be regarded as foreign antigens when the antibody disclosed herein is administered, and thus the antibody molecule exemplified by the present invention, through PD-L1, can stimulate a strong T cell response that is not inhibited by a negative signal.

In some embodiments, the antibody molecule disclosed herein is used to treat and/or prevent inflammatory and autoimmune diseases and graft versus host diseases (GvHD) to down-regulate the immune system.

In other aspects, the present invention provides use of the antibody molecule or the fragment thereof, the immunoconjugate thereof, the combination product or the kit in the manufacture or preparation of a drug in treating the related diseases or conditions mentioned herein.

In some embodiments, the antibody or the fragment thereof, the immunoconjugate, the composition, the combination product or the kit disclosed herein delays the onset of the conditions and/or symptoms associated with the conditions.

In some embodiments, the antibody, the pharmaceutical composition, the immunoconjugate, the combination product or the kits disclosed herein can also be administered in combination with one or more additional therapies, such as therapeutic modalities and/or additional therapeutic agents, for prevention and/or treatment as described herein.

In some embodiments, the treatment modality includes surgery, radiotherapy, partial irradiation, focused irradiation, or the like. In some embodiments, the therapeutic agent is selected from chemotherapeutic agents, cytotoxic agents, vaccines, additional antibodies, anti-infective active agents and immunomodulatory agents.

Exemplary vaccines include, but are not limited to, cancer vaccines. The vaccine may be a DNA-based vaccine, a RNA-based vaccine, or a virus transduction-based vaccine. Cancer vaccines can be prophylactic or therapeutic.

Exemplary anti-infective active agents include, but are not limited to, antivirals, antifungals, antiprotozoals and antibacterials.

In some further embodiments, the antibody or the fragment thereof disclosed herein can also be used in combination with a tyrosine kinase inhibitor.

In some embodiments of any of the methods disclosed herein, the antibody or the fragment thereof disclosed herein is administered in combination with a tumor antigen. The antigen may be, for example, a tumor antigen, a viral antigen, a bacterial antigen, or an antigen from a pathogen. In some embodiments, the tumor antigen comprises a protein. In some embodiments, the tumor antigen comprises a nucleic acid. In some embodiments, the tumor antigen is a tumor cell.

In some embodiments, the antibody or the fragment thereof disclosed herein can be administered in combination with an anti-tumor agent.

In some embodiments, the antibody or the fragment thereof disclosed herein can be administered in combination with a cytokine. The cytokine can be administered as a fusion molecule with the antibody molecule disclosed herein, or as a separate composition. In one embodiment, the antibody disclosed herein is administered in combination with one, two, three or more cytokines (e.g., as a fusion molecule or as a separate composition).

In some embodiments, the antibody or the fragment thereof disclosed herein can be combined with a conventional cancer therapy in the art including, but not limited to, (i) radiotherapies or ionizing radiation to kill cancer cells and shrink tumors, wherein the radiotherapy can be conducted through external beam radiotherapy (EBRT) or internal brachytherapy; (ii) chemotherapies, or applying of cytotoxic drugs, which generally affect cells that are rapidly dividing; (iii) targeted therapies, or agents that specifically affect the deregulation of cancer cell proteins; (iv) immunotherapies, or enhancing of immune responses in a host (e.g., vaccine); (v) hormone therapies, or blocking of hormones (e.g., when the tumors are sensitive to hormones); (vi) angiogenesis inhibitors, or blocking of angiogenesis and growth; and (vii) palliative care, or such therapies that relate to improving the quality of healthcare to manage pain, nausea, vomiting, diarrhea and bleeding to allow a more aggressive therapeutic regimen.

In some embodiments, the antibody or the fragment thereof disclosed herein can be combined with conventional methods for enhancing immune function in a host.

The various combination therapies described above can be further combined for treatment.

Such combination therapies encompass both combined administration (wherein two or more therapeutic agents are contained in the same preparation or separate preparations), and separate administrations (wherein the antibody disclosed herein can be administered prior to, concurrently with, and/or subsequent to the administration of additional therapies, e.g., therapeutic modalities or therapeutic agents). The antibody molecule and/or additional therapies, e.g., therapeutic agents or therapeutic modalities, can be administered when a disease is active or when the disease is in remission or less active. The antibody molecule can be administered prior to, concurrently with, subsequent to other therapies, or during remission of a disease.

In one embodiment, the antibody disclosed herein and additional therapies (such as therapeutic modalities or therapeutic agents) are administered within about one month, or within about one, two or three weeks, or within about 1, 2, 3, 4, 5 or 6 days from each other.

It will be understood that any treatment can be performed using the immunoconjugate, the composition, the combination product or the kit disclosed herein in place of or in addition to the antibody disclosed herein.

The antibody disclosed herein (the pharmaceutical composition or the immunoconjugate comprising the same, and any additional therapeutic agents) can be administered by any suitable method, including parenteral administration, intrapulmonary administration, intranasal administration, and intralesional administration if required by locoregional treatment. Parenteral infusion includes intramuscular, intravenous, intra-arterial, intraperitoneal or subcutaneous administration. The administration is carried out by any suitable means, such as injection, e.g., intravenous or subcutaneous injection, to some extent depending on whether the treatment is short-term or long-term. Various administration schedules are encompassed herein, including, but not limited to, single administration or multiple administrations at multiple time points, bolus injection, and pulse infusion.

In order to prevent or treat diseases, the appropriate dosage of the antibody disclosed herein (when used alone or in combination with one or more other additional therapeutic agents) will depend on types of diseases to be treated, types of antibodies, severity and progression of the disease, purpose of administration (prophylactic or therapeutic) of the antibody, previous treatments, clinical histories of patients, responses to the antibody, and the discretion of an attending physician. The antibody is suitably administered to a patient through a single treatment or through a series of treatments.

The dosage and regimen of the antibody molecule disclosed herein can be determined by those skilled. In some embodiments, the dosage regimen is adjusted to provide the optimal desired response (for example, a therapeutic response). For example, a single bolus injection may be given, several separate doses may be administered over time, or the dosage may be proportionally reduced or increased as indicated by the criticality of the treatment condition. It is particularly advantageous to prepare a parenteral composition in a dosage unit form for ease of dosage administration and uniformity. The dosage unit form as used herein refers to physically separated units suitable as unitary doses for subjects to be treated; each unit contains a predetermined quantity of active compound, which is calculated to produce a desired therapeutic effect in combination with a required pharmaceutical carrier. The specification of the dosage unit form of the present invention is directly dependent on (a) the unique characteristics of the active compound and the particular therapeutic effect to be achieved, and (b) limitations which are unique in the field of combining the active compound for a sensitive therapy in an individual.

### X. Methods and compositions for diagnosis and detection

In certain embodiments, any of the antibodies or the antigen-binding fragments thereof provided herein can be used to detect the presence of an antigen to which it binds in a biological sample. The term "detection" used herein includes quantitative and qualitative detections, and exemplary detections may involve immunohistochemistry, immunocytochemistry, flow cytometry (e.g., FACS), magnetic beads complexed with antibody molecules, ELISA, and PCR (e.g., RT-PCR). In certain embodiments, the biological sample is blood, serum, or other liquid samples of biological origin. In certain embodiments, the biological sample includes cells or tissues. In some embodiments, the biological sample is derived from a hyperproliferative or cancerous lesion.

In one aspect, the present invention provides a diagnostic method for detecting the presence of related antigens in a biological sample, such as serum, semen, urine or tissue biopsy samples (e.g., from a hyperproliferative or cancerous lesion), *in vitro* or *in vivo.* The diagnostic method comprises: (i) contacting a sample (and optionally a control sample) with the antibody molecule as described herein or administering the antibody molecule to a subject under conditions that allow interactions, and (ii) detecting the formation of a complex between the antibody molecule and the sample (and optionally the control sample). The formation of a complex indicates the presence of the related antigen and may show the suitability of or need for the treatment and/or prevention described herein.

In one embodiment, the antibody disclosed herein can be used to diagnose a disease, for example, to assess (e.g., monitor) the treatment or progression, diagnosis and/or staging of the disease (e.g., tumor or infection) described herein in a subject. In certain embodiments, the labeled antibody disclosed herein is provided. The label includes, but is not limited to, a label or moiety that is detected directly, e.g., a fluorescent label, a chromophoric label, an electron-dense label, a chemiluminescent label, and a radioactive label, and a moiety that is detected indirectly, such as an enzyme or a ligand, for example, by enzymatic reaction or molecular interaction. Exemplary labels include, but are not limited to, radioisotopes ³²P, ¹⁴C, ¹²⁵I, ³H and ¹³¹I, fluorophores (such as rare earth chelates or fluorescein) and derivatives thereof, rhodamine and derivatives thereof, dansyl, umbelliferone, luceriferase (such as firefly luciferase and bacterial luciferase (U.S. Patent No. 4,737,456)), fluorescein, 2,3-dihydrophthalazinedione, horseradish peroxidase (HR), alkaline phosphatase, β-galactosidase, glucoamylase, lysozyme, carbohydrate oxidase (such as glucose oxidase, galactose oxidase and glucose-6-phosphate dehydrogenase), heterocyclic oxidase (such as uricase and xanthine oxidase), enzymes oxidizing dye precursors with hydrogen peroxide (such as HR, lactoperoxidase, or microperoxidase), biotin/avidin, spin labels, phage labels, stable free radicals, etc.

In some embodiments of any of the inventions provided herein, the sample is obtained prior to treatment with the antibody disclosed herein. In some embodiments, the sample is obtained after the cancer has metastasized. In some embodiments, the sample is a formalin-fixed, paraffin-embedded (FFPE) sample. In some embodiments, the sample is a biopsy (e.g., a core biopsy) specimen, a surgical specimen (e.g., a specimen from a surgical resection), or a fine-needle aspirate.

In some embodiments, the related antigen is detected prior to the treatment, e.g., prior to the initial treatment or prior to a certain treatment after a treatment interval.

In some embodiments, the level and/or distribution of related antigens are/is determined *in vivo.* For example, the antibody molecule disclosed herein labeled with a detectable substance is detected in a non-invasive manner, e.g., by using appropriate imaging techniques such as positron emission tomography (PET) scanning. In one embodiment, for example, the level and/or distribution of related antigens are/is determined *in vivo* by detecting the antibody molecule disclosed herein that is detectably labeled with a PET reagent (e.g., ¹⁸F-fluorodeoxyglucose (FDG)).

In one embodiment, the present invention provides a diagnostic kit comprising the antibody molecule described herein and an instruction for use.

In some embodiments, provided is a method for treating a disease, wherein the method comprises: detecting the presence of a related antigen in a subject (for example, using a sample, e.g., a sample containing cancer cells of the subject), thereby determining a value; comparing the value to a control value (e.g., the value of the related antigen in a healthy individual); and if the value is greater than the control value, administering to the subject a therapeutically effective amount of the antibody disclosed herein, optionally in combination with one or more of additional therapies, thereby treating the disease.

In some embodiments, the antigen is PD-L1 (e.g., human PD-L1) and/or LAG-3 (e.g., human LAG-3).

It can be understood that the embodiments described in each part of the present invention, such as diseases, therapeutic agents, therapeutic modalities and administration, are equally applicable to, or may be combined with, embodiments of other parts of the present invention. Embodiments described in each part of the present invention that apply to the properties, use and method of the antibody molecule are also applicable to the compositions, conjugates, combination products and kits comprising the antibody.

The following examples are described to assist in understanding the invention. The examples are not intended to be and should not be interpreted in any way as limiting the protection scope of the present invention.

### Examples

### Example 1: Construction of Anti-LAG-3/PD-Ll Bispecific Antibody

In this example, an anti-LAG-3/PD-L1 bispecific antibody having the structure shown in FIG. 1 was constructed, wherein an antigen A was LAG-3 and an antigen B was PD-L1.

Specific sequences of the bispecific antibody are as follows:

| | |
|---|---|
| VL: | SEQ ID NO: 8; |
| VL-CL (i.e., peptide chain #2): | SEQ ID NO: 7; |
| Linker: | SEQ ID NO: 5; |
| VH: | SEQ ID NO: 2; |
| CHI: | SEQ ID NO: 3; |
| Fc (i.e., CH2-CH3): | SEQ ID NO: 4; |
| VHH: | SEQ ID NO: 6; |
| VH-CHI-Fc-VHH (i.e., peptide chain #1): | SEQ ID NO: 1. |

### Example 2. Expression, Purification and Analysis of Anti-LAG-3/PD-Ll Bispecific Antibody

In this example, the nucleotide sequences encoding the peptide chain #1 and peptide chain #2 of the anti-LAG-3/PD-L1 bispecific antibody constructed in Example 1 were linked to the commercially available eukaryotic expression vector pTT5 via multiple cloning sites, and after expression in eukaryotic cells and purification, anti-LAG-3/PD-L1 bispecific antibody IGN-LP was obtained. The specific procedures are as follows.

Genewiz Suzhou was entrusted to synthesize the nucleotide sequences encoding the above chains of the bispecific antibody IGN-LP. The synthesized nucleotide sequences encoding the peptide chains were separately linked to the vector pTT5 using appropriate restriction enzymes and ligases, and recombinant vectors respectively comprising the nucleotide sequences encoding the peptide chains were obtained.

The recombinant vectors were verified to be correct by sequencing and then used for subsequent expression.

HEK293 cells (purchased from Invitrogen) were subcultured in Expi293 cell culture medium (purchased from Invitrogen). The cell culture was centrifuged the day before transfection to obtain a cell precipitate. The cells were suspended in a fresh Expi293 cell culture medium and the cell density was adjusted to 1 × 10⁶ cells/mL. HEK293 cells were further cultured such that the cell density in the culture on the day of transfection was about 2×10⁶ cells/mL.

F17 culture medium (purchased from Gibco, Product Catalog. No. A13835-01) that was 1/10 the final volume of HEK293 cell suspension was used as a transfection buffer. 200 µg of the prepared recombinant plasmids respectively comprising the nucleotide sequences encoding the peptide chain #1 and the peptide chain #2 at a molar ratio of 1:1 was added to each milliliter of the transfection buffer, and the resulting mixture was mixed well. Then 30 µg of polyethylenimine (PEI) (Polysciences, Catalog No.: 23966) was added, and the resulting mixture was mixed well and incubated at room temperature for 10 min, and then the PEI/DNA mixture was gently poured into HEK293 cell suspension. The mixture was mixed well gently and cultured overnight at 8% CO₂ and 36.5 °C.

After overnight incubation, 200 g/L FEED (Sigma, Catalog No.: H6784-100G) that was 1/50 the volume of the culture after transfection and 200 g/L glucose solution that was 1/50 the volume of the culture after transfection were supplemented to the culture flask. The mixture was mixed well gently and cultured at 8% CO₂, 36.5 °C. After 20 h, VPA (Gibco, Catalog No.: 11140-050) was added to a final concentration of 2 mM/L. On day 7 of continuous culturing or when the cell viability was ≤ 60%, the culture was collected and centrifuged at 7500 rpm for 30 min. The cell supernatant was filtered using SARTOPORE (Sartorius, Catalog No.: 5441307H4) and purified by affinity chromatography on an *AKTApure* system (GE Healthcare).

Specific steps for affinity chromatography are as follows:
1) Affinity chromatography: A MabSelect SuRe (GE Healthcare, Catalog No.: 17-5438-03) affinity chromatography column was selected and mounted in the *AKTApure* system. The *AKTApure* system equipped with the MabSelect SuRe affinity chromatography column was sterilized with 0.1 M NaOH overnight. On the day of sample collection, the cells were centrifuged at 7500 rpm for 30 min and filtered by SARTOPORE (Sartorius, 5441307H4).
   The system was washed and the column was equilibrated with 5 column volumes of binding buffer (Tris 20 mM, NaCl 150 mM, pH 7.2) before purification. The filtered cell supernatant was enabled to pass through the column. The column was reequilibrated with 5-10 column volumes of the binding buffer and monitored to UV flatness using the UV detection device in the *AKTApure* system.
   Then, the antibody was eluted with an elution buffer (citric acid + sodium citrate, 100 mM, pH 3.5), and samples were collected based on the UV absorption value. Each 1 mL of the collected solution was neutralized with 80 µL of a neutralization buffer (Tris-HCl, 2M) for late use.
2) Buffer exchange: Collected samples in all the fraction tubes were analyzed for purity by size exclusion chromatography (SEC).

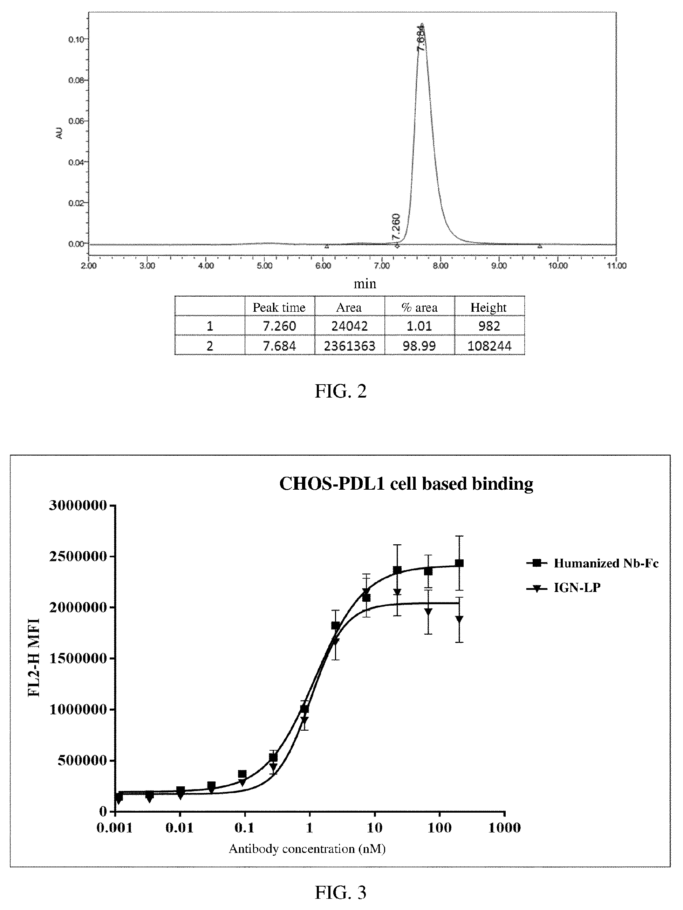

The purified bispecific antibody solution was centrifuged in a 15 mL ultrafiltration centrifuge tube at 4500 rpm for 30 min. The protein was diluted with PBS and further centrifuged at 4500 rpm for 30 min, and this operation was repeated twice to exchange the buffer. The antibodies after buffer exchange were combined, and the antibody concentration was measured. The SEC results are shown in FIG. 2. IGN-LP has a high purity with a main monomer peak purity of 98.99%.

The antibodies obtained were selected for further study in subsequent experiments.

### Example 3. Production and Purification of Anti-LAG-3 Antibody ADI-31853

cDNAs encoding the light and heavy chain amino acid sequences (SEQ ID NO: 7 and SEQ ID NO: 19) of the anti-LAG-3 antibody ADI-31853 were each cloned into an expression vector pTT5 according to a conventional method in the art.

The expression vector containing target antibody genes and a transfection reagent PEI (Polysciences) were transiently transfected into incubated human embryonic kidney cells 293 (Invitrogen) according to a scheme provided by the manufacturer. After transfection, the medium was discarded and the cells were diluted to 4×10⁶/mL with a fresh EXPI293 medium (Gibco). The cells were cultured at 37 °C, 5% CO₂ for 7 days, with fresh medium fed every 48 h. After 7 days, the medium was centrifuged at 1300 rpm for 20 min. The supernatant was purified with Protein A to achieve an antibody purity of greater than 95%.

An anti-PD-Ll humanized Nb-Fc antibody was obtained according to the method described in ZL201710657665.3.

### Example 4. Detection of Binding Activity of Bispecific Antibody to Antigens

The equilibrium dissociation constant (K_{D}) for the binding of the prepared exemplary anti-LAG-3/PD-L1 bispecific antibody IGN-LP disclosed herein to LAG-3 and PD-L1 was determined by a kinetic binding assay using the Octet system manufactured by ForteBio. A ForteBio affinity assay was performed according to the method reported in the literature (Estep, P, et al., High throughput solution Based measurement of antibody-antigen affinity and epitope binning. MAbs, 2013, 5(2): p. 270-278). Briefly:
1) a sensor was provided: the AHQ sensor (Pall, Catalog No.: 1506091) was immersed in a SD buffer (PBS 1×, BSA 0.1%, Tween 20 0.05%) and equilibrated at room temperature half an hour before the experiment.
2) 100 µL of the SD buffer as a blank control (for background subtraction), 100 µL of 100 nM purified bispecific antibody IGN-LP, and anti-PD-Ll humanized Nb-Fc antibody (ZL201710657665.3) and the anti-LAG-3 antibody ADI-31853 (prepared as described in Example 3) as controls, and 100 µL of solutions of human PD-L1-his (100 nM) and human LAG-3-his (100 nM) (Sino Biological) diluted in the SD buffer as antigens were added in a 96-well black polystyrene half-area microplate (Greiner). The anti-human IgG Fc biosensor AHQ was immersed in each well containing the antibody solution at room temperature for 600 s to load the samples. The sensor was then washed in SD buffer until it returned to the baseline, and then immersed in a well containing 100 µL of the antigen solution to monitor the binding of the antibody to the antigen. The sensor was then transferred to a well containing 100 uL of the SD buffer to monitor the antibody dissociation (the operation procedures were set as: Baseline, Loading ~1 nm, Baseline, Association and Dissociation, each running for a time depending on the binding and dissociation rates of the samples). The rotation speed was 400 rpm and the temperature was 30°C. The background-corrected association and dissociation curves were fitted by the Octet analysis software (ForteBio) to generate the association rate constant (kₒₙ) and dissociation rate constant (k_{dis}), which are then used to calculate the equilibrium dissociation constant (K_{D}). The kₒₙ, k_{dis} and K_{D} data of the bispecific antibody IGN-LP and the antigen LAG-3 or PD-L1 are shown in Table 1 and Table 2.

**Table 1. Affinity of anti-PD-L1/LAG-3 bispecific antibody for LAG-3 as determined by ForteBio kinetic binding assay**

| Antibodies | Antibody on AHQ tip/human LAG-3-His in solution (100 nM) [monovalent affinity (M)] | Association constant kₒₙ (1/Ms) | Dissociation constant k_{dis} (1/s) |
|---|---|---|---|
| IGN-LP | 1.71E-09 | 1.17E+05 | 2.00E-04 |
| ADI-31853 | 1.56E-09 | 1.28E+05 | 2.00E-04 |

**Table 2. Affinity of anti-PD-L1/LAG-3 bispecific antibody for PD-L1 as determined by ForteBio kinetic binding assay**

| Antibodies | Antibody on AHQ tip/human PD-L1-His in solution (100 nM) [monovalent affinity (M)] | Association constant kₒₙ (1/Ms) | Dissociation constant k_{dis} (1/s) |
|---|---|---|---|
| IGN-LP | 1.68E-08 | 3.47E+05 | 5.84E-03 |
| Humanized Nb-Fc | 1.15E-08 | 3.10E+05 | 3.56E-03 |

As shown in the above tables, IGN-LP can bind to both PD-L1 and LAG-3 proteins while maintaining the affinity constant of the parent antibody.

### Example 5: Analysis on Binding of Anti-LAG-3/PD-Ll Bispecific Antibody Disclosed Herein to Cells Overexpressing LAG-3 or PD-L1

The binding of the anti-LAG-3/PD-L1 bispecific antibody IGN-LP disclosed herein to cells overexpressing LAG-3 or PD-L1 was determined by FACS.

Briefly, using the ExpiCHO^{™} Expression System Kit (Invitrogen, Catalog No.: A29133), the following operations were carried out according to the manufacturer's instructions:
The pCHO1.0 vector (Invitrogen) carrying human LAG-3 or human PD-L1 cDNA (Sino Biological Inc.) cloned to the multiple cloning site MCS was transfected into HEK293 cells (Invitrogen) and Chinese hamster ovary cancer cells (CHOS) (Invitrogen) to obtain 293 cells overexpressing human LAG-3 (293-LAG-3) and CHOS cells overexpressing PD-L1 (CHO-PD-L1).

To verify whether the bispecific antibody can bind to antigens expressed on the cell surface, the present study detected the binding of the bispecific antibody to 293-LAG-3 cells or CHOS-PD-L1 cells using flow cytometry, and the experimental process is as follows:
1) 293-LAG-3/CHO-PD-L1 cells were counted, and the cell suspension was diluted to 1 × 10⁶ cells/mL with cell medium, and then added to a U-bottom 96-well plate at 100 [µL/well.
2) Detection: The cell suspension was centrifuged at 400 g on a centrifuge for 5 min to remove the cell culture medium. The serial dilutions of the bispecific antibody IGN-LP disclosed herein and anti-humanized Nb-Fc and anti-LAG-3 antibody ADI31853 as controls were added to the U-shaped plate at 100 µL each, and then the cells were resuspended and put onto the ice to stand for 30 min. The cell suspension was centrifuged at 400 g for 5 min to remove the supernatant, and the cells were washed with PBS to remove the unbound antibodies. The resulting cell suspension was centrifuged at 400 g for 5 min to remove PBS. 100 µL of the solution of PE-conjugated anti-human Fc antibody (Jackson Immuno Research) diluted at a ratio of 1:200 was added to each well, and the cells were incubated in the dark on the ice for 30 min. Then the cell suspension was centrifuged at 400 g for 5 min to remove the supernatant. The cells were washed with PBS once to remove the unbound PE-conjugated anti-human Fc antibody. Then the cells were resuspended with 100 µL of PBS, and the binding of the antibody to cells was assayed by FACS.

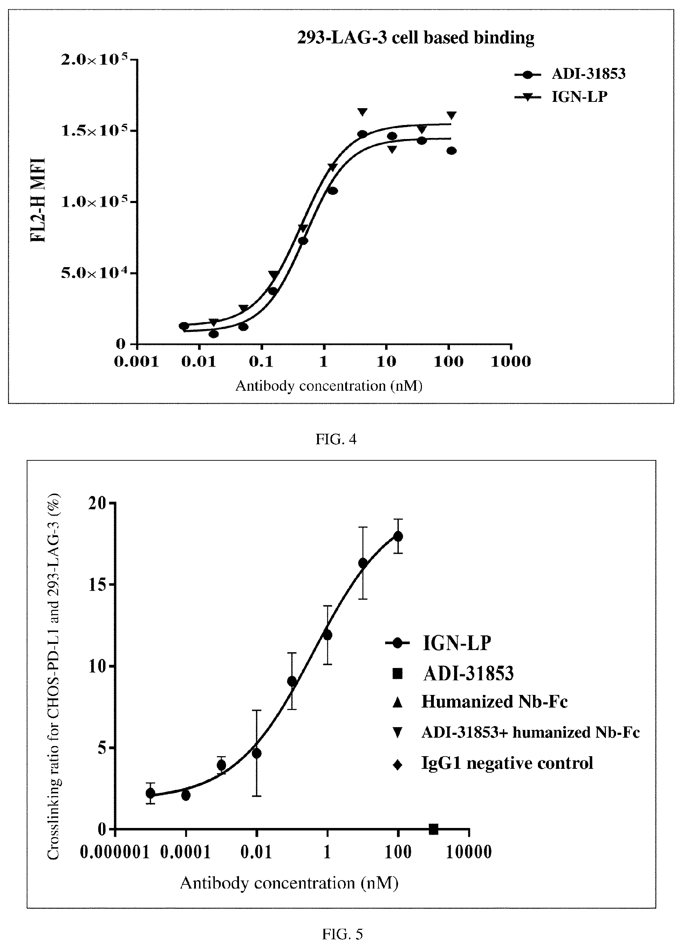

The results are shown in FIG. 3. IGN-LP can bind to PD-L1 on the cell surface at an EC₅₀ of 1.033 nM, which is similar to that of parental anti-PD-Ll antibody (EC₅₀ of 1.199 nM). As shown in FIG. 4, IGN-LP can bind to LAG-3 on the cell surface at an EC₅₀ of 0.4345 nM, which is similar to that of parental anti-LAG-3 antibody ADI-31853 (EC₅₀ of 0.5039 nM).

### Example 6. Analysis on Simultaneous Binding of Anti-LAG-3/PD-Ll Bispecific Antibody Disclosed Herein to 293 Cells Overexpressing LAG-3 and CHO Cells Overexpressing PD-L1

To verify whether the bispecific antibody can bind simultaneously to target antigens from different cells or not, the cross-linking of different cells induced by the bispecific antibody was detected in this example by flow cytometry. The specific experimental process is as follows.
1) CHO-PD-L1 cells and 293-LAG-3 cells were obtained as described in Example 5 and cultured. Cultures containing the CHO-PD-L1 cells and the 293-LAG-3 cells, respectively, were centrifuged at 400 g for 5 min on a centrifuge to remove the cell culture medium. After washed once with PBS, the cells were resuspended in PBS. The cells were counted, and the cell density was adjusted to 2×10⁶ cells/mL. The CHO-PD-L1 cells and the 293-LAG-3 cells were added with CellTracker^{™} Deep Red (Thermo) and Cell Trace CFSE (Invitrogen) dyes at 1:5000, respectively, and incubated at 37 °C for 30 min. The mixture was centrifuged at 400 g for 5 min on a centrifuge to remove the supernatant, and the cells were washed once with PBS.
2) Each of serial dilutions of the bispecific antibody IGN-LP disclosed herein and anti-humanized Nb-Fc (1000 nM) and anti-LAG-3 antibody ADI31853 (1000 nM) as controls were added to a U-bottom 96-well plate. The stained CHO-PD-L1 cells of 1) above were added and mixed (with the final cell density of 1.5×10⁶ cells/mL). The U-bottom 96-well plate was incubated at 4 °C for 30 min, and then the plate was centrifuged at 400 g for 5 min and washed four times with PBS. The cells were then resuspended in PBS.
3) The stained 293-LAG-3 cells of 1) above were added to the cell suspension of 2) above in the U-bottom 96-well plate so that the final density of 293-LAG-3 cells was adjusted to 1×10⁶ cells/mL, and assay was carried out using a flow cytometer (BD, ACCURIC6) after the plate was incubated at room temperature for 1 h. The ratio of double positive cells of channel 2 and channel 4 can reflect the cross-linking of cells caused by the bispecific antibody IGN-LP.

The results are shown in FIG. 5. IGN-LP can induce the cross-linking of CHOS-PD-L1 cells and 293-LAG-3 cells, demonstrating that the bispecific antibody disclosed herein can bind simultaneously to target antigens from different cell surfaces.

The heavy chain (HC) amino acid sequence of the negative control IgG1 used in this example is as follows:

EVRLLESGGGLVQPGGSLRLSCAASGFTFSNYAMGWVRQAPGKGLEWVSAISGSGGS
TYYADSVKGRFTTSRDDSKNALYLQMNSLRAEDTAVYYCARGGPGWYAADVWGQG
TTVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKAEPKSCDKTHTCPP
CPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK (SEQ ID NO: 21);

The light chain (LC) amino acid sequence of the negative control IgG1 is as follows:

DIQMTQSPSSLSASVGDRVTITCRASQSISSYLNWYQQKPGKAPKLLIYAASSLQSGVPS
RFSGSGSGTDFTLTISSLQPEDFATYYCQQADLPAFAFGGGTKVEIKRTVAAPSVFIFPPS
DEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 22).

### Example 7. Thermal Stability detection of Anti-LAG-3/PD-Ll Bispecific Antibody Disclosed Herein

With differential scanning fluorimetry (DSF), information about structure stability can be provided according to the process of fluorescence change in spectrum, and the configuration change of a protein can be detected. The temperature corresponding to the maximum absolute value of the fluorescence curve is the Tm of the protein. In the present study, Tm values of the bispecific antibody disclosed herein are determined using DSF method, and the experimental process is as follows:
1) Antibody sample was diluted to 1 mg/mL with PBS. The SYPRO Orange protein gel stain (Gibco, S6650) was diluted 50-fold with PBS, i.e., 196 µL of PBS was added to 4 µL of the SYPRO Orange protein gel stain stock solution.
2) 50 µL of diluted antibody sample, 10 µL of SYPRO Orange protein gel stain dilution and 40 µL of water were added to a 96-well PCR plate. The plate was detected in a 7500 real time PCR system.

The experimental results are shown in Table 3. The Tm of bispecific antibody is > 63 °C, showing a better thermal stability.

**Table 3: Tm Value of DSF of Bispecific Antibody IGN-LP Disclosed Herein**

| Antibodies | Tm1 | Tm2 |
|---|---|---|
| IGN-LP | 63.5°C | 80.0°C |

### Example 8: Detection of Activation of T cells by Anti-LAG-3/PD-Ll Bispecific Antibody Disclosed Herein

In the present study, the activation of human T cells by the bispecific antibody is detected using a mixed lymphocyte assay, and the specific experimental process is as follows:
1) PBMC isolation: 2.5-fold PBS was added into 50 mL fresh blood from a donor. The mixture was gently added to FiColl (Thermo) and aliquoted into 4 tubes with 12.5 mL each tube. The samples were centrifuged on a centrifuge at 400 g for 30 min before stopping at a deceleration of 0 rpm. The intermediate white strip was pipetted into PBS and washed twice with PBS.
2) DC cell isolation: the isolated PBMC were added with 5 mL of T cell medium (X-VIVO 15 (LONZA)) and then subjected to adherent culture for 2 h at 37 °C, 6% CO₂. The cell suspension was pipetted for CD4+ cell isolation. The remaining cells were added with 3 mL of DC medium, cultured for 2 days, and then added with 3 mL of DC medium (X-VIVO 15 (Lonza) 99%, human AB serum (Access) 1%, HEPES 10 mM, β-Me 50 µM, IL-4 (R&D Systems) 1000 U/mL, and GM-CSF (R&D Systems) 1000 U/mL). On day 5 of culturing, rTNFa (R&D Systems) (1000 U/mL), IL-1b (R&D Systems) (5 ng/mL), IL-6 (R&D Systems) (10 ng/mL) and 1 µM PGE2 (R&D Systems) were added, and the resulting mixture was cultured for 2 days as DC for mixed lymphocyte reaction (MLR).
3) CD4+ cell isolation: the PBMC obtained in 1) were subjected to static culture for 2 h before the cell suspension was pipetted into a 20 mL centrifuge tube. The cells were centrifuged at 200 g for 10 min, and the precipitate was resuspended with 500 µL of a separating medium, 100 µL of AB type serum and 100 µL of purified antibody. The mixture was incubated for 20 min at 4 °C, and washed once with the separating medium (Stemcell (20144)). 500 µL of Bead Buffer was added for an incubation of 15 min, and the beads were then removed by a magnet. The mixture was washed once with a T cell culture medium (X-VIVO 15 (LONZA)), and resuspended with 8 mL of the T cell culture medium. The resulting mixture was then incubated at 37 °C, 6% CO₂. (This was performed according to the instructions of the 'Human CD4+ T cell Enrichment Kit' (19052, Stemcell), for example, the kit was used and all reagents are obtained from the Kit).
4) Bead stimulation for CD4+ cells: CD4+ Cells (1×10⁷) obtained in 3) were resuspended in 4 mL of medium and Dynabeads Human T-Activator CD3/CD28 (Invitrogen) were added at a ratio of 1:1, and the resulting mixture was cultured for three days.
5) MLR: mature DC cells were mixed with stimulated CD4+ cells at 200 mL per well, with 10000 DC and 100000 CD4+ cells, and serially diluted antibodies, 1 nM SEE (Toxin technology) as well as IgG (human IgG (equitech-Bio)) as negative controls were mixed with the mixture and cultured for 3 days.
6) The antibodies used and their starting concentrations are as follows:
   IgG: 10 mg/mL
   IGN-LP: 11.3 mg/mL
   Anti-PD-Ll humanized Nb-Fc antibody: 1.27 mg/mL
   Anti-LAG-3 antibody ADI-31853: 10.5 mg/mL
7) The concentration of IL-2 in the supernatant was measured using the Cisbio Human IL-2 kit.
   Preparation of detection buffer (reagents are all from Human IL-2 1000 tests kit, Cisbio) 30 µL of Human IL-2 d2 antibody (origin) was added to 570 µL of detection buffer (origin), and 30 µL of Human IL-2 cryptate antibody (origin) was added to 570 µL of detection buffer, and the resulting two were mixed at a ratio of 1:1.

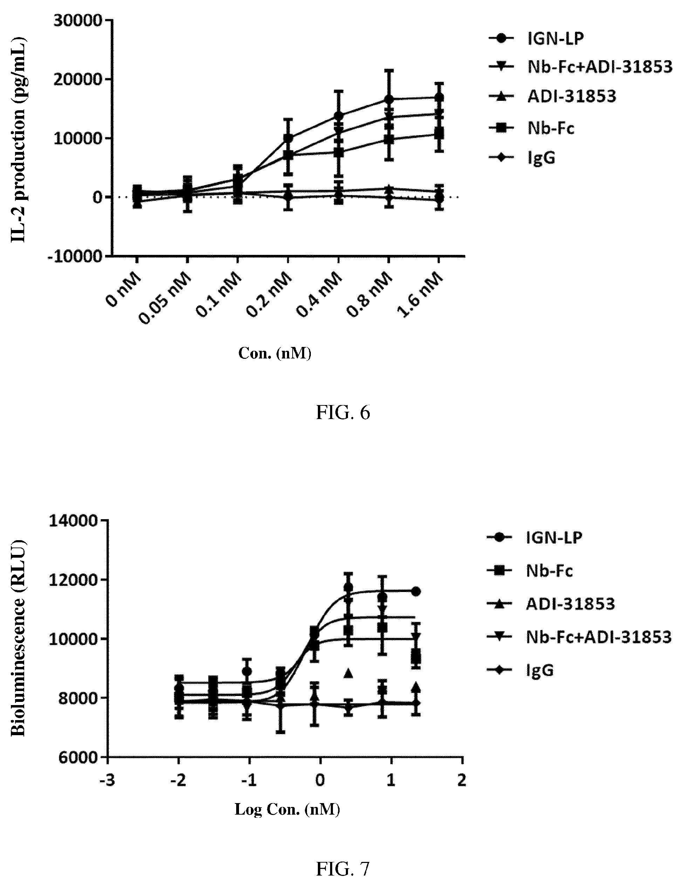

The experimental results are shown in FIG. 6. The bispecific antibody can activate T cells *in vitro,* and its activation effect is stronger than either the anti-PD-Ll antibody or anti-LAG-3 antibody alone, and is similar to the anti-PD-Ll antibody and anti-LAG-3 antibody in combination.

### Example 9. Detection of Ability of Bispecific Antibody to Promote TCR:pMHC Binding

CHOK1-PD-L1 cells (Promega, CS187109) were seeded in a 96-well plate at 4×10⁵/well on day in advance, and 100 uL of RPMI 1640 (Gibco, 22400-071) was provided in each well. On the day of the experiment, Jurkat-LAG3 cells (Promega, (Promega, CS187109)) were counted and the cell density was adjusted to 2.5× 10⁶ cells/mL with culture medium.

IgG (human IgG (equitech-Bio)) as a negative control, the bispecific antibody IGN-LP disclosed herein, the anti-PD-Ll humanized Nb-Fc antibody, the anti-LAG-3 antibody ADI-31853, and Nb-Fc+ADI-31853 were subjected to three-fold gradient dilution with RPMI 1640 (Gibco, 22400-071) from an initial concentration of 200 nM for 10 gradients in total.

The supernatant of CHOK1-PD-L1 was removed from the 96-well plate and the Jurkat-LAG3 cells described above were added at 40 uL/well. Each diluted sample was added at 40 uL/well and incubated at 37 °C for 16 h.

After 16 h, the plate was read using a multifunctional microplate reader (Thermo, Max 13) to obtain bioluminescence (RU) (fluorescent signals) of TCR:pMHC. The concentration-dependent curve was fitted with GraphPad. The assay results are shown in FIG. 7. The bispecific antibody IGN-LP can better promote TCR:pMHC binding and activate downstream signaling pathways than the monoclonal antibody humanized Nb-Fc antibody and anti-LAG-3 antibody ADI-31853 alone or even in combination (humanized Nb-Fc antibody+anti-LAG-3 antibody ADI-31853).

### Example 10. Anti-tumor Activity of Anti-LAG-3/PD-L1 Bispecific Antibody Disclosed Herein

The anti-tumor effect of the anti-LAG-3/PD-L1 antibody IGNLP disclosed herein (i.e., IGN-LP prepared as described in Example 2) was determined using PD-L1 transgenic mice inoculated with MC38-huPD-L1 KI (MJ-1) colon cancer cells (MC 38-huPD-L1 KI tumor-bearing mouse models, abbreviated as MC38/PD-L1 models).

MC38-huPD-L1 KI tumor-bearing mouse models were established by subcutaneous inoculation, and the mice were grouped after tumor formation and treated with different antibodies. Tumor volume and body weight change of each group were monitored during administration, wherein the administration frequency was twice a week for 2 weeks, and the total of 5 doses were given. The mice were monitored twice a week for 4 weeks. Dosages and route of administration are as follows. After the treatment was ended, tumor growth inhibition (TGI%) was calculated by the following formula: TGI% = 100% ∗ (h-IgG control group tumor volume - treatment group tumor volume)/(h-IgG control group tumor volume - h-IgG control group tumor volume before treatment), wherein the average tumor volume of the h-IgG control group was 80 mm³ before treatment.

Mice: PD-L1 transgenic mice, female, 7-8 weeks old (ages at tumor cell inoculation), 18-20 g weight, purchased from Shanghai Model Organisms Center, Inc. The study started after the mice were acclimated for 7 days after arrival.

Cells: human PD-L1 gene (Nanjing Galaxy Biopharma Co., Ltd.) was knocked in the mouse colon cancer cells MC38 (purchased from OBiO Technology (Shanghai) Co., Ltd., HYC0116) to obtain MC38 cells containing human PD-L1, which were cultured in RPMI 1640 medium (Gibco, 22400-071), and subjected to conventional subculture strictly according to MC38 culture requirements for subsequent *in vivo* experiments. The cells were collected by centrifugation (400 g/min, 5 min) and resuspended in sterile RPMI 1640 basic medium, with the cell density adjusted to 5×10⁶ cells/mL. The PD-L1 transgenic mice were subjected to right dorsal shaving and subcutaneous injection of MC38-huPD-L1 KI cells at 0.2 mL/mouse. The tumor volume of each mouse was measured 5 days after inoculation, and mice with the tumor volume ranging from 76-80 mm³ were selected and randomly divided into groups by tumor volume. The anti-tumor activity of the anti-LAG-3/PD-L1 antibody alone was detected by the following administration regimen.

Administration: the mice were divided into 11 groups (6 mice per group), which were injected subcutaneously with the following doses of antibodies, respectively:
(1) human IgG (equitech-Bio), 15 mg/kg;
(2) anti-PD-L1 antibody (humanized anti-PD-L1 antibody Nb-Fc), 1.25 mg/kg;
(3) anti-PD-Ll antibody (humanized anti-PD-Ll antibody Nb-Fc), 2.5 mg/kg;
(4) anti-PD-Ll antibody (humanized anti-PD-Ll antibody Nb-Fc), 5 mg/kg;
(5) LAG-3 (ADI-31853), 10 mg/kg;
(6) LAG-3 (ADI-31853), 10 mg/kg + anti-PD-Ll antibody (humanized anti-PD-Ll antibody Nb-Fc), 1.25 mg/kg;
(7) LAG-3 (ADI-31853), 10 mg/kg + anti-PD-Ll antibody (humanized anti-PD-Ll antibody Nb-Fc), 2.5 mg/kg;
(8) LAG-3 (ADI-31853), 10 mg/kg + anti-PD-Ll antibody (humanized anti-PD-Ll antibody Nb-Fc), 5 mg/kg;
(9) anti-LAG-3/PD-L1 antibody (IGNLP), 3 mg/kg;
(10) anti-LAG-3/PD-L1 antibody (IGNLP), 6 mg/kg;
(11) anti-LAG-3/PD-L1 antibody (IGNLP), 12 mg/kg.

Human IgG was a preparation of human IgG obtained from Equitech-Bio.

On days 5, 9, 12, 15 and 18 after tumor cell inoculation, mice in each group were administered with respective antibody at the dosage described above.

Analysis: the tumor volume and the body weight were measured twice a week throughout the study, and the mice were euthanized when tumors reached an endpoint (the tumor volume was greater than 3000 mm³), or when the mice had more than 20% of weight loss. The maximum length of major axis (L) and maximum length of minor axis (W) of tumors were measured with a vernier caliper, and tumor volume was calculated using the following formula: V = L×W²/2. The tumor volume versus time of each group was plotted. Statistical significance was determined using analysis of variance (ANOVA). P value below 0.05 was considered statistically significant in all analyses.

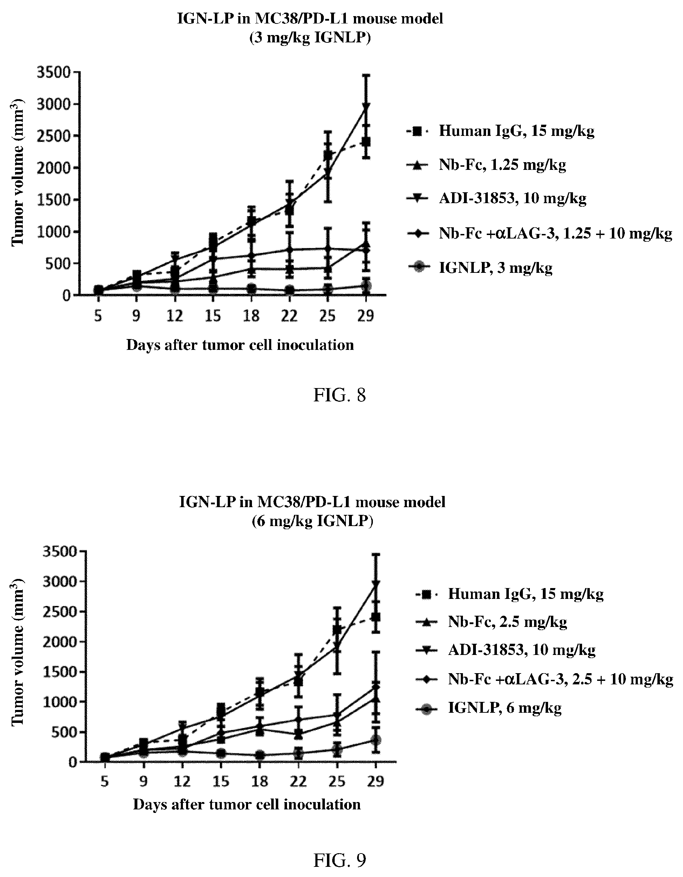

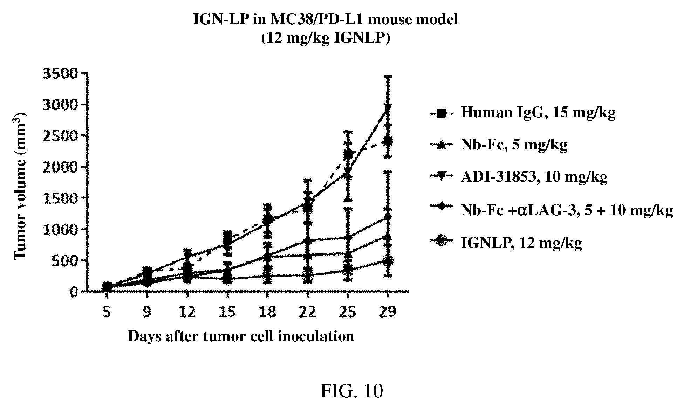

The experimental results are shown in the following table and in FIGs. 8-10. It can be seen that the anti-LAG-3/PD-L1 antibody IGNLP disclosed herein can significantly inhibit tumor growth alone compared to IgG control (equitech-Bio) or anti-LAG-3 antibody and anti-PD-Ll antibody alone or in combination.

**Table 4. Tumor Growth Inhibition on Day 29**

| Groups | Tumor volume | Tumor growth inhibition (%) | Number of mice having complete regression of tumor |
|---|---|---|---|
| Human IgG | 2411 | - | 0/6 |
| Nb-Fc, 1.25 mg/kg | 829 | 68 | 0/6 |
| Nb-Fc, 2.5 mg/kg | 1065 | 58 | 0/6 |
| Nb-Fc, 5 mg/kg | 902 | 65 | 0/6 |
| ADI-31853 | 2942 | - | 0/6 |
| ADI-31853+ Nb-Fc, 1.25 mg/kg | 705 | 73 | 0/6 |
| ADI-31853+ Nb-Fc, 2.5 mg/kg | 1248 | 50 | 0/6 |
| ADI-31853+ Nb-Fc, 5 mg/kg | 1198 | 52 | 0/6 |
| IGNLP, 3 mg/kg | 151 | 97 | 2/6 |
| IGNLP, 6 mg/kg | 367 | 88 | 0/6 |
| IGNLP, 12 mg/kg | 500 | 82 | 1/6 |

**Table 5. Information about sequences**

| Name | Sequence No. |
|---|---|
| IGN-LP peptide chain #1 (exemplary polypeptide chain of formula (I)) | SEQ ID NO: 1 |
| VH of anti-LAG-3 antibody ADI-31853 (exemplary VH of formula (I)) | SEQ ID NO:2 |
| CHI (exemplary CHI of formula (I)) | SEQ ID NO:3 |
| Fc (exemplary Fc of formula (I)) | SEQ ID NO:4 |
| Linker (exemplary X of formula (I)) | SEQ ID NO:5 |
| Anti-PD-Ll single-domain antibody (exemplary VHH of formula (I)) | SEQ ID NO:6 |
| IGN-LP peptide chain #2 (exemplary polypeptide chain of formula (II)) | SEQ ID NO:7 |
| VL of anti-LAG-3 antibody ADI-31853 (exemplary VL of formula (II)) | SEQ ID NO:8 |
| CL (exemplary CL of formula (II)) | SEQ ID NO:9 |
| CDR1 of anti-PD-Ll single-domain antibody (exemplary VHH CDR1 of formula (I)) | SEQ ID NO:10 |
| CDR2 of anti-PD-Ll single-domain antibody (exemplary VHH CDR2 of formula (I)) | SEQ ID NO:11 |
| CDR3 of anti-PD-Ll single-domain antibody (exemplary VHH CDR3 of formula (I)) | SEQ ID NO:12 |
| HCDR1 of anti-LAG-3 antibody ADI-31853 (exemplary HCDR1 of the VH of formula (I)) | SEQ ID NO:13 |
| HCDR2 of anti-LAG-3 antibody ADI-31853 (exemplary HCDR2 of the VH of formula (I)) | SEQ ID NO:14 |
| HCDR3 of anti-LAG-3 antibody ADI-31853 (exemplary HCDR3 of the VH of formula (I)) | SEQ ID NO:15 |
| LCDR1 of anti-LAG-3 antibody ADI-31853 (exemplary LCDR1 of the VL of formula (II)) | SEQ ID NO:16 |
| LCDR2 of anti-LAG-3 antibody ADI-31853 (exemplary LCDR2 of the VL of formula (II)) | SEQ ID NO:17 |
| LCDR3 of anti-LAG-3 antibody ADI-31853 (exemplary LCDR3 of the VL of formula (II)) | SEQ ID NO:18 |
| Heavy chain of anti-LAG-3 antibody (exemplary VH-CH1-Fc of formula (I)) | SEQ ID NO:19 |

**Table 6. Sequences**

| SEQ ID NO | Sequences |
|---|---|
| 1 | QLQLQESGPGLVKPSETLSLTCTVSGGSIYSESYYWGWIRQPPGKGLEWIGSIVYSGYTY YNPSLKSRVTISVDTSKNQFSLKLSSVTAADTAVYYCARVRTWDAAFDIWGQGTMVTV SSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPGGGGGSGGGGSQVQLQESGGGL VQPGGSLRLSCAASAYTISRNSMGWFRQAPGKGLEGVAAIESDGSTSYSDSVKGRFTISL DNSKNTLYLEMNSLRAEDTAVYYCAAPKVGLGPRTALGHLAFMTLPALNYWGQGTLV TVSS |
| 2 | QLQLQESGPGLVKPSETLSLTCTVSGGSIYSESYYWGWIRQPPGKGLEWIGSIVYSGYTY YNPSLKSRVTISVDTSKNQFSLKLSSVTAADTAVYYCARVRTWDAAFDIWGQGTMVTV SS |
| 3 | ASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHT |
| 4 | CPPCPAPEAAGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG |
| 5 | GGGGSGGGGS |
| 6 | QVQLQESGGGLVQPGGSLRLSCAASAYTISRNSMGWFRQAPGKGLEGVAAIESDGSTS YSDSVKGRFTISLDNSKNTLYLEMNSLRAEDTAVYYCAAPKVGLGPRTALGHLAFMTL PALNYWGQGTLVTVSS |
| 7 | DIQMTQSPSSLSASVGDRVTITCQASQDISNYLNWYQQKPGKAPKLLIYDASNLETGVPS RFSGSGSGTDFTFTISSLQPEDIATYYCQQVLELPPWTFGGGTKVEIKRTVAAPSVFIFPPS DEQLKSGTASVVCLLNNFYPREAKVQWKVDNALQSGNSQESVTEQDSKDSTYSLSSTL TLSKADYEKHKVYACEVTHQGLSSPVTKSFNRGEC |
| 8 | DIQMTQSPSSLSASVGDRVTITCQASQDISNYLNWYQQKPGKAPKLLIYDASNLETGVPS RFSGSGSGTDFTFTISSLQPEDIATYYCQQVLELPPWTFGGGTKVEIK |
| 9 | |
| 10 | AYTISRNSMG (AbM rule) |
| 11 | AIESDGSTSYSDSVKG (Kabat rule) |
| 12 | PKVGLGPRTALGHLAFMTLPALNY (Kabat rule) |
| 13 | GSIYSESYYWG |
| 14 | SIVYSGYTYYNPSLKS (Kabat rule) |
| 15 | ARVRTWDAAFDI (IMGT rule) |
| 16 | QASQDISNYLN (Kabat rule) |
| 17 | DASNLET (Kabat rule) |
| 18 | QQVLELPPWT (Kabat rule) |
| 19 | QLQLQESGPGLVKPSETLSLTCTVSGGSIYSESYYWGWIRQPPGKGLEWIGSIVYSGYTY YNPSLKSRVTISVDTSKNQFSLKLSSVTAADTAVYYCARVRTWDAAFDIWGQGTMVTV SSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPG |

## Claims

1. An antibody molecule or an antigen-binding fragment thereof, comprising or consisting of:
(i) a polypeptide chain of formula (I):
VH-CH1-Fc-X-VHH;
and
(ii) a polypeptide chain of formula (II):
VL-CL;
wherein:
the VH represents a heavy chain variable region;
the CH represents a heavy chain constant region;
the Fc comprises CH2, CH3, and optionally CH4;
the CH1, the CH2, the CH3 and the CH4 represent domains 1, 2, 3 and 4, respectively, of the heavy chain constant region;
the X may be absent, or represents a linker when present;
the VHH represents a single-domain antigen-binding site;
the VL represents a light chain variable region;
the CL represents a light chain constant region;
optionally, a hinge region is present between the CHI and the Fc.

2. The antibody molecule or the antigen-binding fragment thereof according to claim 1, comprising or consisting of 1 or 2 polypeptide chains of formula (I) and 1 or 2 polypeptide chains of formula (II).

3. The antibody molecule or the antigen-binding fragment thereof according to claim 1 or 2, wherein the linker is a flexible linker, and preferably the linker comprises an amino acid sequence (Gly₄Ser)n, wherein n is a positive integer equal to or greater than 1, e.g., n is a positive integer from 1 to 7, such as 1,2, 3, 4, 5 or 6.

4. The antibody molecule or the antigen-binding fragment thereof according to any one of claims 1-3, wherein the antibody or the fragment thereof is a human antibody or a humanized antibody, or a chimeric antibody.

5. The antibody molecule or the antigen-binding fragment thereof according to any one of claims 1-4, wherein the single-domain antigen-binding site (VHH) is a heavy chain variable domain of an antibody naturally devoid of light chains, e.g., a heavy chain variable domain of a heavy chain antibody naturally existing in a Camelidae species, or a VH-like single domain in an immunoglobulin of fish referred to as a new antigen receptor (e.g., IgNAR naturally existing in shark serum), or a recombinant single-domain antigen-binding site derived therefrom (e.g., a camelized human VH domain or a humanized Camelidae antibody heavy chain variable domain); preferably, the single-domain antigen-binding site is selected from a heavy chain variable domain of a heavy chain antibody naturally existing in a Camelidae species, a camelized human VH domain and a humanized Camelidae antibody heavy chain variable domain.

6. The antibody molecule or the antigen-binding fragment thereof according to any one of claims 1-5, wherein the "CH1-Fc" of the formula (I) is in the form of IgG, e.g., IgG1, IgG2 or IgG4, and/or the CL of the formula (II) is from κ or λ

7. The antibody molecule or the antigen-binding fragment thereof according to any one of claims 1-6, wherein an antigen-binding site formed by the VH and VL is specific for a first antigen and an antigen-binding site formed by the VHH is specific for a second antigen; preferably, the first antigen is the same as or different from the second antigen.

8. The antibody molecule or the antigen-binding fragment thereof according to claim 7, wherein the first antigen and the second antigen are selected from a cytokine, a growth factor, a hormone, a signaling protein, an inflammatory mediator, a ligand, a cell surface receptor or a fragment thereof, a tumor-associated antigen, an immune checkpoint molecule, an angiogenic factor, a member of a tumor necrosis factor receptor superfamily, a co-stimulatory molecule in immune system, and ligands and/or receptors thereof, such as OX40, CD47, PD1, PD-L1, PD-L2, LAG-3, 4-1BB (CD137), VEGF and GITR.

9. The antibody molecule or the antigen-binding fragment thereof according to claim 7, wherein the first antigen is LAG-3, e.g., human LAG-3; and/or wherein the second antigen is PD-L1, e.g., human PD-L1.

10. The antibody molecule or the antigen-binding fragment thereof according to any one of claims 1-9, wherein the VHH in the formula (I):
(i) comprises three complementarity determining regions (VHH CDRs) in SEQ ID NO: 6; or
(ii) comprises complementarity determining regions (CDRs) VHH CDR1, VHH CDR2 and VHH CDR3, wherein the VHH CDR1 comprises or consists of an amino acid sequence set forth in SEQ ID NO: 10, the VHH CDR2 comprises or consists of an amino acid sequence set forth in SEQ ID NO: 11, and the VHH CDR3 comprises or consists of an amino acid sequence set forth in SEQ ID NO: 12; or
(iii) comprises or consists of a sequence set forth in SEQ ID NO: 6; or
(iv) comprises or consists of an amino acid sequence having at least 90%, 91%, 92%, 93%, 94%, 95%, 96%, 97%, 98% or 99% identity to an amino acid sequence set forth in SEQ ID NO: 6.

11. The antibody molecule or the antigen-binding fragment thereof according to any one of claims 1-10, wherein
the VH in the formula (I):
(i) comprises three complementarity determining regions HCDRs of a heavy chain variable region VH set forth in SEQ ID NO: 2; or
(ii) comprises complementarity determining regions (CDRs) HCDR1, HCDR2 and HCDR3, wherein the HCDR1 comprises or consists of an amino acid sequence set forth in SEQ ID NO: 13, the HCDR2 comprises or consists of an amino acid sequence set forth in SEQ ID NO: 14, and the HCDR3 comprises or consists of an amino acid sequence set forth in SEQ ID NO: 15; or
(iii) comprises or consists of an amino acid sequence set forth in SEQ ID NO: 2; or
(iv) comprises or consists of an amino acid sequence having at least 90%, 91%, 92%, 93%, 94%, 95%, 96%, 97%, 98% or 99% identity to an amino acid sequence set forth in SEQ ID NO: 2;
and/or
the VL:
(i) comprises three complementarity determining regions LCDRs of a heavy chain variable region VL set forth in SEQ ID NO: 8; or
(ii) comprises complementarity determining regions (CDRs) LCDR1, LCDR2 and LCDR3, wherein the LCDR1 comprises or consists of an amino acid sequence set forth in SEQ ID NO: 16, the LCDR2 comprises or consists of an amino acid sequence set forth in SEQ ID NO: 17, and the LCDR3 comprises or consists of an amino acid sequence set forth in SEQ ID NO: 18; or
(iii) comprises or consists of an amino acid sequence set forth in SEQ ID NO: 8; or
(iv) comprises or consists of an amino acid sequence having at least 90%, 91%, 92%, 93%, 94%, 95%, 96%, 97%, 98% or 99% identity to an amino acid sequence set forth in SEQ ID NO: 8.

12. The antibody molecule or the antigen-binding fragment thereof according to any one of claims 1-11, wherein
(a) the VH-CH1-Fc in the formula (I):
(i) comprises or consists of an amino acid sequence having at least 85%, 90%, 91%, 92%, 93%, 94%, 95%, 96%, 97%, 98% or 99% identity to an amino acid sequence set forth in SEQ ID NO: 19; or
(ii) comprises or consists of an amino acid sequence set forth in SEQ ID NO: 19;
and/or
(b) the VL-CL of the formula (II):
(i) comprises or consists of an amino acid sequence having at least 85%, 90%, 91%, 92%, 93%, 94%, 95%, 96%, 97%, 98% or 99% identity to an amino acid sequence set forth in SEQ ID NO: 7; or
(ii) comprises or consists of an amino acid sequence set forth in SEQ ID NO: 7.

13. The antibody molecule or the antigen-binding fragment thereof according to any one of claims 1-12, wherein the polypeptide chain of formula (I) comprises or consists of a sequence set forth in SEQ ID NO: 1 or an amino acid sequence having at least 85%, 90%, 91%, 92%, 93%, 94%, 95%, 96%, 97%, 98% or 99% identity thereto; and/or wherein the polypeptide chain of formula (II) comprises or consists of a sequence set forth in SEQ ID NO: 7 or an amino acid sequence having at least 85%, 90%, 91%, 92%, 93%, 94%, 95%, 96%, 97%, 98% or 99% identity thereto.

14. An isolated nucleic acid encoding any one or more polypeptide chains of the antibody molecule or the antigen-binding fragment thereof according to any one of claims 1-13.

15. A vector comprising the nucleic acid according to claim 14, wherein, preferably, the vector is an expression vector, such as a pTT5 vector.

16. A host cell, comprising the nucleic acid according to claim 14 or the vector according to claim 15, wherein, preferably, the host cell is prokaryotic or eukaryotic; more preferably, the host cell is selected from an *E. coli* cell, a yeast cell, a mammalian cell and other cells suitable for preparation of the antibody or the antigen-binding fragment thereof; most preferably, the host cell is a 293 cell or a CHO cell.

17. A method for preparing the antibody molecule or the antigen-binding fragment thereof according to any one of claims 1-13, comprising culturing the host cell according to claim 16 under conditions suitable for expressing the nucleic acid according to claim 14, and optionally isolating the antibody or the antigen-binding fragment thereof, wherein optionally, the method further comprises isolating the antibody or the antigen-binding fragment thereof from the host cell.

18. An immunoconjugate, comprising the antibody molecule or the antigen-binding fragment thereof according to any one of claims 1-13 and an additional substance, e.g., a therapeutic agent or a label, such as a cytotoxic agent.

19. A pharmaceutical composition, comprising the antibody molecule or the antigen-binding fragment thereof according to any one of claims 1-13 or the immunoconjugate according to claim 18, and optionally a pharmaceutical supplementary material.

20. A pharmaceutical composition, comprising the antibody molecule or the antigen-binding fragment thereof according to any one of claims 1-13 or the immunoconjugate according to claim 17 and an additional therapeutic agent, and optionally a pharmaceutical supplementary material, wherein, preferably, the additional therapeutic agent is selected from a chemotherapeutic agent, an additional antibody, a cytotoxic agent, a vaccine, an anti-infective active agent, a small molecule drug and an immunomodulatory agent.

21. A combination product, comprising the antibody molecule or the antigen-binding fragment thereof according to any one of claims 1-13 or the immunoconjugate according to claim 18, and one or more additional therapeutic agents, such as a chemotherapeutic agent, a cytotoxic agent, a vaccine, an additional antibody, an anti-infective active agent, a small molecule drug, or an immunomodulatory agent.

22. A method for preventing or treating a disease in a subject, comprising administering to the subject an effective amount of the antibody molecule or the antigen-binding fragment thereof according to any one of claims 1-13, the immunoconjugate according to claim 18, the pharmaceutical composition according to claim 19 or 20, or the combination product according to claim 21, wherein the disease is, for example, an autoimmune disease, an inflammatory disease, an infection, or a tumor; for example, the tumor is a cancer, e.g., a cancer with elevated expression levels of PD-1, PD-L1 or PD-L2 and/or LAG-3, e.g., colon cancer or colorectal cancer or rectal cancer.

23. The method according to claim 22, further comprising co-administering to the subject one or more additional therapies, wherein the therapy, for example, comprises a therapeutic modality and/or an additional therapeutic agent; preferably, the therapeutic modality comprises surgical treatment and/or radiotherapy, or the therapeutic agent is selected from a chemotherapeutic agent, a cytotoxic agent, a vaccine, an additional antibody, an anti-infective active agent, a small molecule drug and an immunomodulatory agent.

24. A method for detecting an antigen in a sample, comprising
(a) contacting the sample with the antibody or the antigen-binding fragment thereof according to any one of claims 1-13; and
(b) detecting a complex formed by the antibody or the antigen-binding fragment thereof and the antigen, wherein, optionally, the antibody is detectably labeled.

25. The method according to claim 24, wherein the antigen is selected from a cytokine, a growth factor, a hormone, a signaling protein, an inflammatory mediator, a ligand, a cell surface receptor or a fragment thereof, a tumor-associated antigen, an immune checkpoint molecule, an angiogenic factor, a member of a tumor necrosis factor receptor superfamily, a co-stimulatory molecule in immune system, and ligands and/or receptors thereof, such as OX40, CD47, PD1, PD-L1, PD-L2, LAG-3, 4-1BB (CD137), VEGF and GITR; preferably, the antigen is PD-L1 and/or LAG-3, such as human PD-L1 and/or human LAG-3.


SEQUENCE LISTING

<110>  陜湛汜昜桊狻(劼笣)岴癹鼠侗

<120>  陔倰祁枅袟俶蕨极煦赽眕摯肮奀賦磁 PD-L1 睿 LAG-3 腔祁枅袟俶蕨极

<130>  PF 190842PCT

<160>  19

<170>  PatentIn version 3.3

<210>  1
<211>  591
<212>  PRT
<213>  Artificial

<220>
<223>  Synthetic constructs

<400>  1

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Tyr Ser Glu
            20                  25                  30

Ser Tyr Tyr Trp Gly Trp Ile Arg Gln Pro Pro Gly Lys Gly Leu Glu
        35                  40                  45

Trp Ile Gly Ser Ile Val Tyr Ser Gly Tyr Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Arg Thr Trp Asp Ala Ala Phe Asp Ile Trp Gly Gln
            100                 105                 110

Gly Thr Met Val Thr Val Ser Ser Ala Ser Thr Lys Gly Pro Ser Val

115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Gly Gly Gly Gly Ser Gly Gly Gly Gly Ser Gln Val Gln Leu Gln
    450                 455                 460

Glu Ser Gly Gly Gly Leu Val Gln Pro Gly Gly Ser Leu Arg Leu Ser
465                 470                 475                 480

Cys Ala Ala Ser Ala Tyr Thr Ile Ser Arg Asn Ser Met Gly Trp Phe
                485                 490                 495

Arg Gln Ala Pro Gly Lys Gly Leu Glu Gly Val Ala Ala Ile Glu Ser
            500                 505                 510

Asp Gly Ser Thr Ser Tyr Ser Asp Ser Val Lys Gly Arg Phe Thr Ile
        515                 520                 525

Ser Leu Asp Asn Ser Lys Asn Thr Leu Tyr Leu Glu Met Asn Ser Leu
    530                 535                 540

Arg Ala Glu Asp Thr Ala Val Tyr Tyr Cys Ala Ala Pro Lys Val Gly
545                 550                 555                 560

Leu Gly Pro Arg Thr Ala Leu Gly His Leu Ala Phe Met Thr Leu Pro
                565                 570                 575

Ala Leu Asn Tyr Trp Gly Gln Gly Thr Leu Val Thr Val Ser Ser
            580                 585                 590

<210>  2
<211>  120
<212>  PRT
<213>  Artificial

<220>
<223>  Synthetic constructs

<400>  2

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Tyr Ser Glu
            20                  25                  30

Ser Tyr Tyr Trp Gly Trp Ile Arg Gln Pro Pro Gly Lys Gly Leu Glu
        35                  40                  45

Trp Ile Gly Ser Ile Val Tyr Ser Gly Tyr Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Arg Thr Trp Asp Ala Ala Phe Asp Ile Trp Gly Gln
            100                 105                 110

Gly Thr Met Val Thr Val Ser Ser
        115                 120

<210> 3
<211> 108
<212> PRT
<213> Artificial

<220>
<223> Synthetic constructs

<400> 3

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr
            100                 105

<210> 4
<211> 221
<212> PRT
<213> Artificial

<220>
<223>  Synthetic constructs

<400>  4

Cys Pro Pro Cys Pro Ala Pro Glu Ala Ala Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Asp Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly
    210                 215                 220

<210>  5
<211>  10
<212>  PRT
<213>  Artificial

<220>
<223>  Synthetic constructs

<400>  5

Gly Gly Gly Gly Ser Gly Gly Gly Gly Ser
1               5                   10

<210>  6
<211>  132
<212>  PRT
<213>  Artificial

<220>
<223>  Synthetic constructs

<400>  6

Gln Val Gln Leu Gln Glu Ser Gly Gly Gly Leu Val Gln Pro Gly Gly
1               5                   10                  15

Ser Leu Arg Leu Ser Cys Ala Ala Ser Ala Tyr Thr Ile Ser Arg Asn
            20                  25                  30

Ser Met Gly Trp Phe Arg Gln Ala Pro Gly Lys Gly Leu Glu Gly Val
        35                  40                  45

Ala Ala Ile Glu Ser Asp Gly Ser Thr Ser Tyr Ser Asp Ser Val Lys
    50                  55                  60

Gly Arg Phe Thr Ile Ser Leu Asp Asn Ser Lys Asn Thr Leu Tyr Leu

65                  70                  75                  80

Glu Met Asn Ser Leu Arg Ala Glu Asp Thr Ala Val Tyr Tyr Cys Ala
                85                  90                  95

Ala Pro Lys Val Gly Leu Gly Pro Arg Thr Ala Leu Gly His Leu Ala
            100                 105                 110

Phe Met Thr Leu Pro Ala Leu Asn Tyr Trp Gly Gln Gly Thr Leu Val
        115                 120                 125

Thr Val Ser Ser
    130

<210>  7
<211>  215
<212>  PRT
<213>  Artificial

<220>
<223>  Synthetic constructs

<400>  7

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Val Leu Glu Leu Pro Pro
                85                  90                  95

Trp Thr Phe Gly Gly Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 8
<211> 108
<212> PRT
<213> Artificial

<220>
<223> Synthetic constructs

<400> 8

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Val Leu Glu Leu Pro Pro
                85                  90                  95

Trp Thr Phe Gly Gly Gly Thr Lys Val Glu Ile Lys
            100                 105

<210>  9
<211>  107
<212>  PRT
<213>  Artificial

<220>
<223>  Synthetic constructs

<400>  9

<210> 10
<211> 10
<212> PRT
<213> Artificial

<220>
<223> Synthetic constructs

<400> 10

Ala Tyr Thr Ile Ser Arg Asn Ser Met Gly
1               5                   10

<210> 11
<211> 16
<212> PRT
<213> Artificial

<220>
<223> Synthetic constructs

<400> 11

Ala Ile Glu Ser Asp Gly Ser Thr Ser Tyr Ser Asp Ser Val Lys Gly
1               5                   10                  15

<210> 12
<211> 24
<212> PRT
<213> Artificial

<220>
<223> Synthetic constructs

<400> 12

Pro Lys Val Gly Leu Gly Pro Arg Thr Ala Leu Gly His Leu Ala Phe
1               5                   10                  15

Met Thr Leu Pro Ala Leu Asn Tyr

            20

<210> 13
<211> 11
<212> PRT
<213> Artificial

<220>
<223> Synthetic constructs

<400> 13

Gly Ser Ile Tyr Ser Glu Ser Tyr Tyr Trp Gly
1               5                   10

<210> 14
<211> 16
<212> PRT
<213> Artificial

<220>
<223> Synthetic constructs

<400> 14

Ser Ile Val Tyr Ser Gly Tyr Thr Tyr Tyr Asn Pro Ser Leu Lys Ser
1               5                   10                  15

<210> 15
<211> 12
<212> PRT
<213> Artificial

<220>
<223> Synthetic constructs

<400> 15

Ala Arg Val Arg Thr Trp Asp Ala Ala Phe Asp Ile
1               5                   10

<210> 16
<211> 11
<212> PRT
<213> Artificial

<220>

<223>  Synthetic constructs

<400>  16

Gln Ala Ser Gln Asp Ile Ser Asn Tyr Leu Asn
1               5                   10


<210>  17
<211>  7
<212>  PRT
<213>  Artificial

<220>
<223>  Synthetic constructs

<400>  17

Asp Ala Ser Asn Leu Glu Thr
1               5


<210>  18
<211>  10
<212>  PRT
<213>  Artificial

<220>
<223>  Synthetic constructs

<400>  18

Gln Gln Val Leu Glu Leu Pro Pro Trp Thr
1               5                   10


<210>  19
<211>  449
<212>  PRT
<213>  Artificial

<220>
<223>  Synthetic constructs

<400>  19

Gln Leu Gln Leu Gln Glu Ser Gly Pro Gly Leu Val Lys Pro Ser Glu
1               5                   10                  15


Thr Leu Ser Leu Thr Cys Thr Val Ser Gly Gly Ser Ile Tyr Ser Glu

            20                  25                  30

Ser Tyr Tyr Trp Gly Trp Ile Arg Gln Pro Pro Gly Lys Gly Leu Glu
        35                  40                  45

Trp Ile Gly Ser Ile Val Tyr Ser Gly Tyr Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Arg Thr Trp Asp Ala Ala Phe Asp Ile Trp Gly Gln
            100                 105                 110

Gly Thr Met Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly